# EUROPEAN PATENT SPECIFICATION

(11) **EP 2 603 222 B1**
(45) Date of publication and mention of the grant of the patent: **17.10.2018**
(21) Application number: 11815949.0
(22) Date of filing: 11.08.2011
(51) Int. Cl.: A61K 31/706, A61K 31/7056, A61P 35/00, A61P 35/02, C07H 19/056, C07H 19/12, A61K 45/06

(54) **COMBINATORY CANCER TREATMENT**
KOMBINATORISCHE KREBSBEHANDLUNG
THÉRAPIE ANTICANCÉREUSE COMBINATOIRE

(30) Priority: 11.08.2010 CA 2712747
(43) Date of publication of application: 19.06.2013
(73) Proprietor: UNIVERSITE DE MONTREAL, Montreal, Quebec H3T 1J4 (CA)
(72) Inventor: BORDEN, Katherine, Dollard des Ormeaux, Québec H9A 3G3 (CA); CULJKOVIC, Biljana, Montreal, Québec H3V 1H3 (CA)
(74) Representative: Gill, Siân Victoria
(86) International application number: PCT/CA2011/000908
(87) International publication number: WO 2012/019284

(56) References cited:
- ZHU XIONGPENG ET AL: "Novel agents and regimens for acute myeloid leukemia: 2009 ASH annual meeting highlights", JOURNAL OF HEMATOLOGY & ONCOLOGY, BIOMED CENTRAL LTD, LONDON UK, vol. 3, no. 1, 23 April 2010 (2010-04-23), page 17, XP021083343, ISSN: 1756-8722, DOI: 10.1186/1756-8722-3-17
- B C KRALJACIC ET AL: "Inhibition of eIF4E with ribavirin cooperates with common chemotherapies in primary acute myeloid leukemia specimens", LEUKEMIA, vol. 25, no. 7, 1 April 2011 (2011-04-01), pages 1197-1200, XP055089775, ISSN: 0887-6924, DOI: 10.1038/leu.2011.57
- S. ASSOULINE ET AL: "Molecular targeting of the oncogene eIF4E in acute myeloid leukemia (AML): a proof-of-principle clinical trial with ribavirin", BLOOD, vol. 114, no. 2, 11 May 2009 (2009-05-11), pages 257-260, XP055089784, ISSN: 0006-4971, DOI: 10.1182/blood-2009-02-205153
- TAN K ET AL: "Ribavirin targets eIF4E dependent Akt survival signaling", BIOCHEMICAL AND BIOPHYSICAL RESEARCH COMMUNICATIONS, ACADEMIC PRESS INC. ORLANDO, FL, US, vol. 375, no. 3, 24 October 2008 (2008-10-24), pages 341-345, XP025398752, ISSN: 0006-291X, DOI: 10.1016/J.BBRC.2008.07.163 [retrieved on 2008-08-14]
- ZHU ET AL.: 'Novel agents and regimens for acute myeloid leukemia: 2009 ASH annual meeting highlights' JOURNAL OF HEMATOLOGY AND ONCOLOGY vol. 3, 2010, page 17, XP021083343
- BORDEN ET AL.: 'Ribavirin as an anti-cancer therapy: acute myloid leukemia and beyond?' LEUKEMIA & LYMPHOMA vol. 51, no. 1, 2010, pages 1805 - 1815
- Paul W. Hollenbach ET AL: "A Comparison of Azacitidine and Decitabine Activities in Acute Myeloid Leukemia Cell Lines", PLoS ONE, vol. 5, no. 2, 2 February 2010 (2010-02-02), page e9001, XP055387740, DOI: 10.1371/journal.pone.0009001
- Fadi Hariri: "The Eukaryotic Translation Initiation Factor eIF4E, a Recently Identified Therapeutic Target In AML, Is a Direct Transcriptional Target of NFkB | Blood Journal", Blood, 19 November 2010 (2010-11-19), XP055387864, Retrieved from the Internet: URL:http://www.bloodjournal.org/content/11 6/21/3146 [retrieved on 2017-07-04]
- HANFENG GUAN ET AL: "Decitabine represses osteoclastogenesis through inhibition of RANK and NF-kB", CELLULAR SIGNALLING., vol. 27, no. 5, 1 May 2015 (2015-05-01), pages 969-977, XP055387872, GB ISSN: 0898-6568, DOI: 10.1016/j.cellsig.2015.02.006

## Description

### BACKGROUND OF THE INVENTION

The invention relates to the treatment of neoplasms (i.e. cancers), proliferative diseases, preneoplasms and precancerous lesions, by a novel combinatory cancer treatment, comprising an inhibitor of eIF4E and a methyltransferase inhibitor.

Cancer is a devastating disease that affects countless persons and families worldwide. One class of cancer is leukemia which consists of malignancies derived from hematopoietic (blood-forming) cells. Part of this class of cancers is acute myeloid leukemia (AML), also known as acute myelogenous leukemia, which is a cancer of the myeloid line of blood cells, characterized by the rapid growth of abnormal white blood cells that accumulate in the bone marrow and interfere with the production of normal blood cells. AML is the most common acute leukemia affecting adults, and its incidence increases with age.

In order to treat patients diagnosed with cancer, scientific researchers around the world have investigated a multitude of mutant cancer cells, genetic mutations, site-specific mutagenesis, DNA, RNA, RNA and protein expression, transporters, genetic sequencing, so as to map biochemical pathways in cancer cells at the molecular level and find the "cure" to various types of cancer. One of the more recent cancer research fields consists of the investigation of the dysregulation of the RNA metabolism that contributes to cells becoming cancerous, and even more specifically, the inhibition of a specific factor, eukaryotic translation initiation factor 4E (eIF4E), by a well known anti-viral drug, Ribavirin, which impedes eIF4E's ability to make cells cancerous without significantly affecting normal cells.

The eukaryotic translation initiation factor, eIF4E, is found in all cells, is a key effector of epigenomic regulation and is important to make new proteins. eIF4E coordinately and combinatorially modules the expression of genes involved in proliferation and survival. The amount of eIF4E is overexpressed in AML, and is abnormally high in 30% of cancers, including breast, prostate and some leukemias, including the particularly aggressive subtypes of myeloid leukemia referred to as M4 and M5. The function of eIF4E to make new proteins depends upon its ability to bind to the front part of RNA known as the m⁷G cap (7-methyl guanosine) (located on the 5' end of the mRNA), which then allows the cell to "translate" or turn this RNA into protein. It also has a role in the export of the mRNA into the cytoplasm, which must precede the translation step. eIF4E levels, in cancer patients, become elevated for many reasons, namely: gene amplification, transcriptional dysregulation, as well as alterations in mRNA stability. Notably, in most cancers, both the mRNA export and translation functions of eIF4E are dysregulated. In the context of cancer, a more detailed discussion on eIF4E can be found in: Borden et al. "Structural characterization of the Z ring-eIF4E complex reveals a distinct mode of control for eIF4E" PNAS, vol. 107(12), p. 5441-5446 (March 23rd, 2010); Borden et al., "Understanding and targeting the eukaryotic translation initiation factor eIF4E in head and neck cancer" Journal of Oncology, 2009 (Epub 2009 Dec. 13, Review); Borden, "Tissue targeting in cancer: eIF4E's tale", Clin Cancer Res, 15(13) p. 4254-4255 (July 1st, 2009); Borden et al. "Stability of eukaryotic translation initiation factor 4E mRNA is regulated by HuR, and this activity is dysregulated in Cancer", Molecular and Cellular Biology, vol. 29 (5), p. 1152-1162 (Mar 2009); Tamburini et al., "Targeting translation in acute myeloid leukemia: a new paradigm for therapy?" Cell Cycle, 8 (23) 3893-3899 (Dec 2009); Borden et al., "Controlling gene expression through RNA regulons: the role of the eukaryotic translation initiation factor eIF4E" Cell Cycle, 6; 65-69 (2007); Borden et al., "eIF4E is a central node of an RNA regulon that governs cellular proliferation", The Journal of Cell Biology, 175 (3), p. 415-426 (Nov 6, 2007); Montanaro et al., "Initiation of mRNA translation in oncogenesis: The role of eIF4E", Cell Cycle, 3:11, 1387-1389 (Nov 2004); Graff et al., "Translational control and metastatic progression: enhanced activity of the mRNA cap-binding protein eIF-4E selectivity enhances translation of metastasis-related mRNAs", Clin Exp Metastasis, 20: 265-273 (2003); and Borden et al. "The emerging roles of translation factor eIF4E in the nucleus", Differentiation, 70: 10-22 (2002); and DeFatta et al., "Antisense RNA to eIF4E suppresses oncogenic properties of a head and neck squamous cell carcinoma cell line", Laryngoscope, 110 (6): 928-933 (Jun 2000). In sum, it is thought that cancer cells with elevated levels of eIF4E seem to have developed an oncogene addiction to eIF4E. For information purposes reference can be made to the following documents, namely: International laid-open publication nos. WO 2007/123579 and WO 2008/060369 (Translational Therapeutics); International laid-open publication no. 2010/006291 (Nodality Inc.); and U.S. Patent Nos. 7,425,544 and 7,601,700 (issued to Eli Lilly and Co. and ISIS Pharmaceuticals Inc.), as well as Canadian Patent Application No. 2,632,903 (Nabil-Habib Lab and Vianova Labs Inc.).

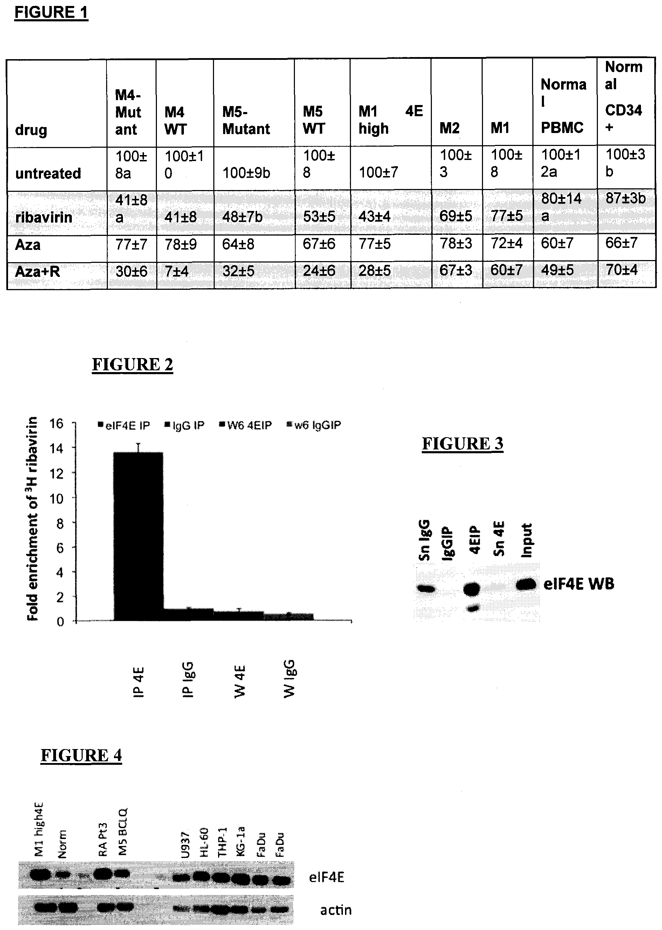

Thus, because of its properties, the eukaryotic translation initiation factor, eIF4E, has become an appealing clinical target to treat patients diagnosed with cancer, in particular AML. In this connection, targeting of the eIF4E-m⁷G cap-binding activity has been studied in a phase II trial, in leukemia patients, and has been reported in Assouline et al., "Molecular targeting of the oncogene eIF4E in acute myeloid leukemia (AML): a proof-of-principle clinical trial with ribavirin" Blood, vol. 114, no. 2 (July 9th, 2009, Epub 2009 May 11). In this trial, the commonly used anti-viral drug, ribavirin, was found to decrease the function of eIF4E because it mimics the m⁷G cap; thus inhibiting eIF4E-induced export and translation of sensitive transcripts. In cell culture experiments, ribavirin did not modulate the levels of eIF4E protein or RNA. However in patients, ribavirn not only inhibits eIF4E, it also can lead to the downregulation of eIF4E protein (and RNA) levels as observed in patients in a phase II clinical trial using ribavirin monotherapy. Finally, in living cells, we demonstrate that eIF4E binds ³H ribavirin (figures 2 and 3) further supporting the idea that eIF4E binds ribavirin directly *in vitro* and *in vivo*.

For the purpose of convenience, ribavirin is chemically designated as:1-[(2*R*,3*R*,4*S*,5*R*)-3,4-dihydroxy-5-(hydroxymethyl)oxolan-2-yl]-1*H*-1 ,2,4-triazole-3-carboxamide, and has the following chemical structure:

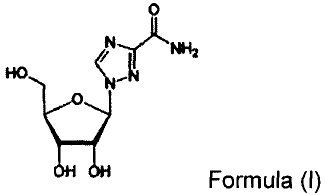

Its preparation has been disclosed in United States Patent No. 3,798,209 (issued to ICN) as well as in the Journal of Medicinal Chemistry, 15, 1150 (1972), Witkowski, J.T., et al. A review of its mechanism of action, in the context of a viral treatment, is disclosed in Streeter et al., "Mechanism of action of 1-β-D-ribofuranosyl-1,2,4-triazole-3-carboxamide (Virazole), A new broad-spectrum anti-viral agent", Proc. Nat. Acad. Sci., vol. 70(4), pp. 1174-1178, (Apr 1973), as well as in the 1972 Science publication, entitled "Broad-spectrum antiviral activity of Virazole: 1-beta-D-ribofuranosyl-1,2,4-triazole-3-carboxamide", authored by Sidwell.

The clinical pharmacology of ribavirin is also disclosed in "The clinical pharmacology of ribavirin" Seminars in liver disease, vol. 19, suppl. 1, 1999, p. 17-24, 1999 by Paul Glue. A further discussion on the interaction of ribavirin with eIF4E can be found in: Assouline et al., "Molecular targeting of the oncogene eIF4E in acute myeloid leukemia (AML): a proof-of-principle clinical trial with ribavirin" Blood, vol. 114, no. 2 (July 9th, 2009); Borden et al., "Tissue Targeting in Cancer: eIF4E's Tale" Clin Cancer Res, 2009, 15(13): 4254-5 (July 1st, 2009); Borden et al. "Ribavirin targets eIF4E dependent Akt survival signalling", Biochem Biophys Res Commun, 375(3): 341-345 (Oct 24, 2008); Borden et al. "Further evidence that ribavirin interactions with eIF4E", RNA, 11:1762-1766 (2005); and Borden et al. "Ribavirin suppresses eIF4E-mediated oncogenic transformation by physical mimicry of the 7-methyl guanosine mRNA cap", PNAS, vol. 101(52) p. 18105-18110 (Dec 28th, 2004). From these disclosures, it can be understood that the physical mimic of the natural ligand of eIF4E, ribavirin, preferentially inhibits the growth of primary AML (M4/M5 AML) specimens with elevated eIF4E levels relative to specimens with normal levels of eIF4E (e.g., M1/M2 AML) or normal controls. It is also taught that when ribavirin monotherapy is used, no treatment-related toxicities are observed. Moreover, in Borden et al., "Further evidence that ribavirin interacts with eIF4E" RNA (2005) 11, 1762-1766, the authors further confirmed that ribavirin antagonized eIF4E functions in transport and translation of eIF4E-sensitive mRNAs at low micromolar concentrations, similar to those at which it associates with purified eIF4E in vitro (Kd in the low micromolar range; see Kentsis et al., "Ribavirin suppresses eIF4E-mediated oncogenic transformation by physical mimicry of the 7-methyl guanosine mRNA cap", PNAS, 2004, Vol. 101(52), pp. 18105-18110). Further studies indicate that ³H ribavirin immunoprecipitates (IPs) with eIF4E in living cells further support the claim that ribavirin directly binds eIF4E (Figures 2 and 3).

In conducting clinical trial no. NCT00559091, the Applicant observed that most if not all patients became resistant to ribavirin monotherapy. In some patients, monotherapy had no impact. Thus, a problem associated with a ribavirin monotherapy, in the context of cancer and as noted in Assouline et al., (*supra*), is that AML cells become resistant to this therapy. In fact, leukemic cells become resistant to nearly all monotherapies within two (2) to four (4) months; such being due to naturally occurring phenomena such as selective evolution (Abboud et al., "Induction therapy for elderly patients with acute myeloid leukemia", Blood Reviews (2008) 22: 311-320, and Melnick et al., "MDS and secondary AML display unique patterns and abundance of aberrant DNA methylation", Blood. 2009 Oct 15;114(16):3448-58. Epub 2009 Aug 3). To overcome this issue of resistance, it is not uncommon in the clinical field, and as for most treatments involving a monotherapy, to follow-up this treatment with chemotherapy, as it taught in Abboud et al. *(supra*) and Melnick et al. (*supra*). As it is well known, the use of chemotherapeutic agents has many secondary effects on patients, including and not limited to damage of normal cells, anemia, bleeding, constipation, fatigue, hair loss, infections, memory changes, swelling, and even death amongst many others. Conventional chemotherapy also requires a stay at the hospital so as to administer the chemotherapeutic agent(s) as well as supportive care for the side effects. In this connection, Abboud et al., in Blood Reviews (2008, supra), review treatment strategies of patients over 60 (which are the majority of AML patients), diagnosed with AML, and the morbidities and mortalities associated with chemotherapy.

Another drug known for treating cancer is azacitidine (AZA, also known as "azacytidine"), which is chemically designated as 4-amino-1-β-D-ribofuranosyl-1,3,5-triazin-2(1*H*)-one and it has the following chemical structure:

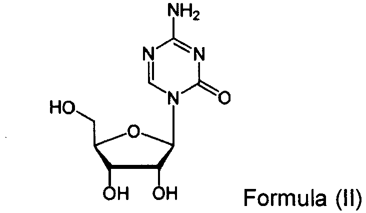

AZA is a prodrug with activity exerted through RNA and DNA. By binding to RNA or its incorporation therein, it causes inhibition of protein synthesis. It is also a cell specific cytotoxic agent and has multiple effects on DNA metabolism. Indeed, when AZA is incorporated into DNA, it is known to produce a marked decrease in the activity of DNA methyltransferase. AZA, a ribonucleoside, is also proven to have an effect on reducing cell viability, in AML cell lines, as disclosed by Hollenback et al., in "A comparison of azacitidine and decitabine activities in acute myeloid leukemia cell lines" PloS ONE, vol. 5, issue 2, Feb 2010. A further discussion of its mechanism of action can be found in Borthakur et al., "Report of a phase 1/2 study of azacitidine and cytarabine in acute myelogenous leukemia and high-risk myelodyplastic syndromes" Leukemia and Lymphoma 2010 January; 51(1); 73-78; Raza et al., "Combination of 5-azacytidine and thalidomide for the treatment of myelodysplatic syndromes and acute myeloid leukemia", Cancer, Oct 2008, vol. 113(7), 1596-1604; Sudan et al. in "Treatment of acute myelogenous leukemia with outpatient azacitidine", Cancer, Oct 15 2006, vol. 107(8), p. 1839-1843; Keating et al., "Azacitidine: a review of its use in higher-risk myelodysplastic syndromes/acute myeloid leukaemia" Drugs, 2009; 69(17): 2501-18; and Garcia-Manero G. "A pilot pharmacokinetic study of oral azacitidine", Leukemia. 2008 Sep; 22(9): 1680-4. Epub 2008 Jun 12. Reference can also be made to U.S. Patent No. 6,905,669, which discloses, in part the methyltransferase inhibitors.

To treat patients diagnosed with cancer, including, for example, AML, many combinations of drugs have been developed and reported in the prior art. For example, Zhu et al., "Novel agents and regime for acute myeloid leukemia: 2009 ASH annual meeting highlights" Journal of Hematology & Oncology 2010, 3:17 (Review) discloses monotherapies of daunorubicin, voreloxin, ARRY-520, AZD1152, AZD6244 and terameprocol, as well as combinations of drugs such as: (i) cytarabine with daunorubicin; (ii) fludarabine, cytarabine with idarubicin, (iii) mitoxantrone with cytarabine; (iv) clofarabine alone or in combination with low-dose Ara-C or high dose Ara-C with the monoclonal antibody GO; (v) combination therapy with sorafenib; (vi) tipifarnib with bortezomib; (vii) vorinostat in combination with idarubicin; (viii) decitabine with GO; (ix) azacitidine with botezomib or low-dose GO; (x) amonafile with Ara-C; (xi) behanoylara-C with idarubicin; (xii) lenalidomine, Ara-C and daunorubicin; as well as (xiii) ribavirin with Ara-C, in the treatment of elderly AML or relapsed AML. The combination of ribavirin and low-dose Ara-C, Ara-C and idarubicin, and combinations thereof (i.e. ribavirin, Ara-C and idarubicin) as well as sorafenib with ribavirin was specifically disclosed by Assouline et al. in a poster session entitled "Targeting the oncogene eIF4E with ribavirin: a novel therapeutic avenue in acute myeloid leukemia" Blood (ASH Annual Meeting abstracts) 2009, vol. 114: Abstract 2085. The combination of ribavirin with sorafenib is also known and was disclosed by W.H. Miller et al. in a Cancer meeting in February 2010, in a PowerPoint presentation entitled "Targeting the protein translation factor eIF4E with Ribavirin: A novel therapeutic avenue in Human Cancer". Other combinatory drug therapies include the combination of inhibitors of DNA methylation with other drugs, such as those reported, for example, in: Leone et al., "Inhibitors of DNA methylation in the treatment of hematological malignancies and MDS", Clinical Immunology, 109 (1), pgs. 89-102 (October 2003); Gore S.D., "Combination therapy with DNA methyltransferase inhibitors in hematologic malignancies", Nature Clinical Practice Oncology (2005) 2, S30-S35, doi:10.1038/ncponc0346 (Accepted 5 September 2005); Brown et al., "Novel targeted drug therapies for the treatment of childhood acute leukemia." Expert Rev Hematol. 2009 Apr 1;2(9):145-158; Sekeres MA., "Treatment of MDS: something old, something new, something borrowed..." Hematology Am Soc Hematol Educ Program. 2009:656-63; and Ma et al., "Novel agents on the horizon for cancer therapy" CA Cancer J Clin. 2009 Mar-Apr;59(2):111-37.

Clinical trials have also been conducted on several combinations of drugs for the treatment of leukemia and/or AML, and are available at: http://clinicaltrials.gov/ct2/home. Examples of combinations of therapy for AML, include and are not limited to: ABT-348; ABT-888 and topotecan with or without carboplatin; alemtuzumab, busulfan, and cyclophosphamide; alemtuzumab, busulfan, and melphalan; alemtuzumab with fludarabine phosphate; all-trans retinoic acid with bryostatin 1; amifostine trihydrate, cytarabine with mitoxantrone hydrochloride; arsenic trioxide; azacitidine with cytarabine arabinoside (also referred to as Ara-C); azacitidine, asparaginase, cytarabine, daunorubicin hydrochloride, etoposide, lintuzumab with thioguanine; azacitidine with arsenic trioxide; azacitidine with belinostat; azacitidine with entinostat; azacitidine with gemtuzumab ozogamicin; azacitidine with lenalidomide; azacitidine with midostaurin ; 5-azacytidine (vidaza®) with panobinostat (lbh589); 5-azacytidine (5-aza), valproic acid with all-trans retinoic acid (atra); azacytidine with valproic acid; azacitidine with phenylbutyrate ; basiliximab; becatecarin; belinostat; bendamustine; bevacizumab, cytarabine with mitoxantrone hydrochloride; bexarotene and gm-csf; BMS-214662; bortezomib with belinostat; bortezomib with melphalan; bortezomib and vorinostat ; bryostatin 1; busulfan, filgrastim with etoposide; busulfan with fludarabine; busulfan, cyclophosphamide, mycophenolate mofetil with tacrolimus; carboplatin, docetaxel with ifosfamide; cediranib maleate; clofarabine; clofarabine with cyclophosphamide; clofarabine, cytarabine with idarubicin; clofarabine, filgrastim with cytarabine; clofarabine and high-dose melphalan; clofarabine, melphalan, and thiotepa; cilengitide; cixutumumab with temsirolimus; CPX-151; CT53518; cytarabine and daunorubicin with or without gemtuzumab ozogamicin; cytarabine and daunorubicin with or without zosuquidar trihydrochloride; cytarabine, idarubicin with tipifarnib; cytarabine with 7-hydroxystaurosporine; cytarabine with laromustine; cytarabine with tanespimycin; cytarabine with triapine; cyclophosphamide; cyclosporine and Given IV with mycophenolate mofetil; cyclosporine, mycophenolate mofetil, and pentostatin; cyclosporine, methotrexate, methoxsalen, mycophenolate mofetil with pentostatin; decitabine; decitabine with lenalidomide; decitabine with romidepsin; decitabine with tretinoin; decitabine with valproic acid; decitabine with vorinostat (sequential); deferasirox; dolastatin 10; eltrombopag olamine; entinostat; everolimus; exatecan mesylate; fentanyl citrate; flavopiridol and vorinostat ; fludarabine and cyclophosphamide as well as total-body irradiation, followed by cyclosporine and mycophenolate mofetil; fludarabine phosphate with Given IV; fludarabine phosphate with tretinoin; fludarabine, carboplatin, and topotecan; fludarabine, carboplatin, topotecan with thalidomide; fludarabine with melphalan; fludarabine with thiotepa; fludarabine with treosulfan; gimatecan; 7-hydroxystaurosporine with perifosine; hydroxyurea with laromustine; idarubicin with saha (vorinostat); ipilimumab; imatinib mesylate; interleukin-12 followed by interferon alfa ; irofulven; itraconazole with midostaurin; ispinesib; JNJ-26481585; KW-2449; laromustine; lintuzumab; lonafarnib; MEK inhibitor AZD6244; MS-275 and gm-csf ; MGCD0103; MLN8237; mycophenolate mofetil, tacrolimus with daclizumab; ON 01910.na; OXI4503; palivizumab with or without ribavirin; paricalcitol; phenylbutyrate and tretinoin ; procrit; pyroxamide; fluorouracil, leucovorin calcium, and topotecan hydrochloride; rasburicase; revlimid; romidepsin; sargramostim, amifostine trihydrate, carboplatin with cyclophosphamide; SB1518; SJG-136; STA-9090; sirolimus with tacrolimus; sodium salicylate; sorafenib tosylate; sorafenib with vorinostat; tacrolimus and mycophenolate mofetil with or without sirolimus ; tacrolimus and mycophenolate mofetil; tetradecanoylphorbol acetate; temsirolimus; tipifarnib; triapine with fludarabine phosphate; vorinostat; and yttrium y 90 anti-cd45 monoclonal antibody ahn-12, amongst others. Raza et al., in "Combination of 5-azacytidine and thalidomide for the treatment of myelodysplatic syndromes and acute myeloid leukemia", Cancer, Oct 2008, vol. 113(7): 1596-1604, discloses the combination of 5-azacytidine and thalidomide, the latter being a drug that was once prohibited because of its teratogenic effects.

As noted by Borthakur et al. in "Report of a phase 1/2 study of azacitidine and cytarabine in acute myelogenous leukemia and high-risk myelodyplastic syndromes" Leukemia Lymphoma 2010 January; 51(1); 73-78, a problem associated with the particular combination of azacitidine and cytarabine is that, even if deemed feasible, it only has limited activity in relapsed/refractory AML. They noted that in the advanced AML population, it was difficult to deliver more than one cycle of therapy, and anti-leukemia activity may be observed with relapsed/refractory disease. As per Borthakur et al., another problem associated to AML drug therapies is epigenetic silencing; a phenomenon by which a drug-induced increased methylation allows for acquired drug resistance. The contribution of epigenetic mechanisms for correct cell function is highlighted by the effects of their deregulation that, in cooperation with genetic alterations, lead to the establishment and progression of tumors (see Fazi et al., "Heterochromatic gene repression of the retinoic acid pathway in acute myeloid leukemia", Blood, May 2007, vol. 109(10), p. 4432-4440). Other problems with concomitant drug therapy is that the drugs may (i) produce antagonistic effects, (ii) undergo collateral sensitivity/resistance to other drugs, (iii) be difficult to determine the right dosing regimen, (iv) have toxicity issues; and (v) create multiple drug resistance. Lastly, AML cells have shown to be capable of self-renewal (see Wang et al. in "Sensitivity to 5-azacytidine blast progenitors in acute myeloblastic leukemia", Blood, vol. 69(2) Feb 1997, p. 553-559).

From the above, it becomes apparent that the treatment of myelodyplastic syndromes (MDS) and/or AML remains a challenge to the clinician despite recent advances. Many patients either will not respond or will have only limited and/or brief responses to single agent therapy or even concomitant therapy. There is thus a need to overcome the aforementioned drawbacks by a novel combinatory drug therapy. In this connection, the present invention is directed to a particular combination of azacitidine (AZA) and ribavirin, which overcomes, for the most part, the aforementioned drawbacks.

The only piece of scientific literature which alludes to ribavirin and azacytidine is a publication entitled "A single mutation in poliovirus RNA-dependent RNA polymerase confers resistance to mutagenic nucleotide analogs via increased fidelity" PNAS, Jun 10, 2003, vol. 100(12), p. 7289-7294. In this publication, the authors, Pfeiffer and Kirkegaard, describe, in a viral environment (i.e. in the poliovirus), the generation of ribavirin-resistant poliovirus by serial viral passage in the presence of increasing concentrations of the drug, ribavirin. The authors noted that the ribavirin resistance can be caused by a single amino acid change, G54S, in the viral polymerase in an unresolved portion of the fingers domain. In comparing with the wild-type virus, the authors noted that ribavirin-resistant poliovirus displays increased fidelity of RNA synthesis in the absence of ribavirin and increased survival both in the presence of ribavirin and another mutagen, 5-azacytidine. Even though this publication alludes to the mere presence of ribavirin and azacytidine, it is not relevant for the following reasons. Firstly, most virus studies have primarily focused on the effects of ribavirin on the virus, for example: mutations in viral polymerases, which is not the case in the context of the present invention. In the viral context, ribavirin impedes growth of the virus and resistance occurs when the virus continues to replicate even in the presence of ribavirin. In the cancer context, it is a measure of cells becoming resistant to the antiproliferative effects of ribavirin, i.e. that eIF4E mediates proliferation, ribavirin impedes this effect and then eventually, the cells continue to proliferate even in the presence of ribavirin. Secondly, there could be different biochemical pathway modulated. Thus, one cannot compare viral infections, such as the hepatitis C virus (HCV) or the poliovirus, with cancerous type cells or cell growth as the mechanisms of action are totally different.

For the same reasons, the scientific publication entitled "Ribavirin resistance in Hepatitis C virus replicon-containing cell lines conferred by changes in the cell line or mutations in the replicon RNA", Journal of Virology, vol. 79(4) Feb 2005, p. 2346-2355, cannot be considered applicable to the present invention.

### SUMMARY OF THE INVENTION

An aspect of the present invention is directed to a pharmaceutical composition comprising a ribavirin, a methyltransferase inhibitor and a pharmaceutically acceptable carrier for use in the treatment of a pre-neoplasm, a precancerous lesion, neoplasm or a proliferative disorder, wherein the methyltransferase inhibitor is azacytidine.

Further embodiments are set out in the dependent claims.

Other features and advantages of the invention will be apparent from the following detailed description, the drawings and the claims.

### BRIEF DESCRIPTION OF THE DRAWINGS

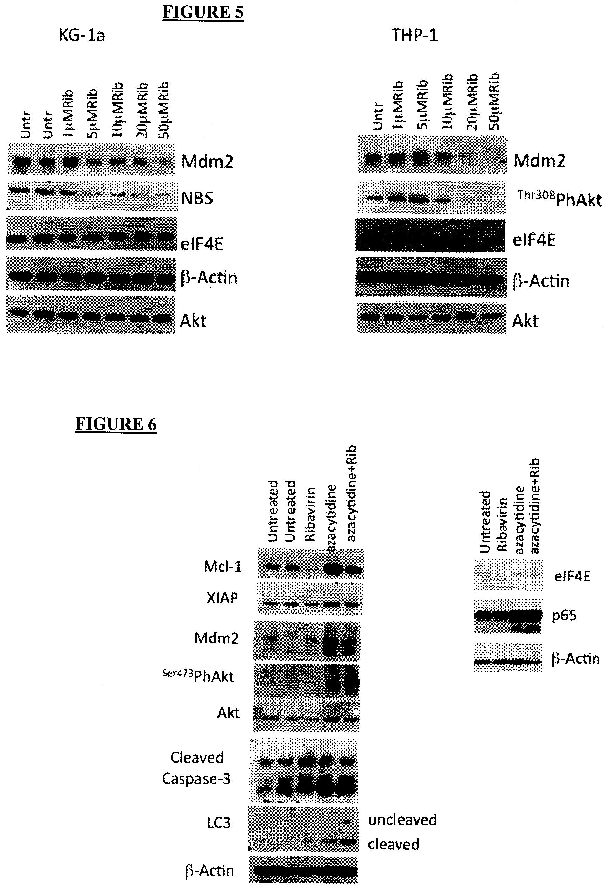

Figure 1 is a table summarizing the results of colony forming assays in methyl cellulose for primary AML specimens (purchased from the BLCQ) or specimens isolated from healthy volunteers (purchased from Stem Cell Technologies). Colony number indicates the proliferative potential of the cells being treated with the drugs given in the Table. A colony experiment typically runs for 14 days. A special media which supports hematopoietic cell growth was used (Methocult purchased from Stem Cell Technologies). Mutant refers to the presence of a mutation in the Flt3 gene (a common leukemia marker) and wildtype refers to Flt3 not being mutated in this specimen. Each experiment is carried out in quintuplicate with colony number plus/minus standard deviations given. Drug treatments for the inhibitor of eIF4E (e.g., ribavirin) were 1 micromolar final concentration replenished every 48 hours (due to the breakdown of ribavirin) and 3 micromolar of methyltransferase inhibitor (e.g. azacytidine), which was not replenished. Untreated controls were carried out in parallel for every single experiment. Experiments were done on specimens as described. Note that AML M1-high4E refers to a M1 specimen that had elevated eIF4E levels (see Figure 5).
Figure 2 is a diagram demonstrating that an inhibitor of eIF4E, e.g., ribavirin, binds directly to eIF4E in living cells. Previous biophysical experiments showed that ribavirin could bind to eIF4E directly by using NMR, mass spectrometry, fluorescence, among other techniques (see Kentsis et al, 2004 (*supra*) and Kentsis, et al., "Further evidence that ribavirin interacts with eIF4E", RNA. 2005 Dec;11(12): 1762-6). To demonstrate that this mechanism is occurring in living cells, FaDu cells were treated 24h with a 0.66 micromolar solution of an inhibitor of eIF4E, e.g., ³H Ribavirin, cross-linked with formaldehyde, and lysed in immunoprecipitation buffer. Equal amounts of lysate were used for immunoprecipitation with antibodies to eIF4E or IgG controls. After 6 washes (W), beads were eluted in SDS Buffer. 1/5 of volume was used for western blot analysis and the rest for ³H measurement. Sn refers to supernatant (the material that did not bind to the antibody-beads i.e. did not immunoprecipitate). Input is a positive control showing that eIF4E is present in the cell lysate.
Figure 3 is a western blot control for Figure 2. This western blot confirms that antibodies to eIF4E immunoprecipitated eIF4E and that eIF4E was not found in the IgG control immunoprecipitation.
Figure 4 is a western blot demonstrating that THP1 and KG1 cells have elevated eIF4E protein levels. The specimen referred to in Figure 1 as M1 high4E is also shown. Actin is provided as a loading control. "Norm" refers to cells isolated from a healthy volunteer. It is worth noting that the intensity of the actin is much higher for the Norm than for all the other specimens bar M1 high4E indicating that the levels of eIF4E are much lower in the normal control than in any of the cell lines or specimens examined relative to actin. FaDu cell lines are provided as a positive control given that they are established to have elevated eIF4E. M5 BCLQ is an AML specimen from the Quebec Leukemia Cell Bank (BCLQ), which is another positive control. RA patient 3 (RA Pt3) refers to the before specimen for one of the patients participating in the Applicant's clinical trial, also clearly has elevated eIF4E. One concludes from this blot that the THP-1 and KG-1a cells have elevated eIF4E levels within the range of other positive specimens.
Figure 5 is a western blot demonstrating that KG-1 and THP-1 cells respond to the inhibitor of eIF4E, e.g., ribavirin, and is thus a good surrogate system to parallel studies in primary patient specimens. Cells were treated with a concentration of an inhibitor of eIF4E (e.g., ribavirin) given for 48 hours. THP-1 cells represent an M4 AML cell line. However, KG-1a cells are an M2 cell line that happens to have elevated eIF4E (see Figure 5). These serve as a parallel for the Applicant's M1-high4E primary specimens. Despite the lineage differences, KG-1a and THP-1 cells respond similarly to the inhibitor of eIF4E (e.g., ribavirin, see Figure 5) and to the combination of inhibitor of eIF4E (e.g., ribavirin) with a methyltransferase inhibitor (e.g., azacytidine, see below). The eIF4E downstream mRNA export target mdm2 is downregulated by the inhibitor of eIF4E, e.g., ribavirin, treatment in both cell lines. eIF4E protein levels do not change, except there is a reduction at 50 uM ribavirin in THP-1 cells, but this is likely to be an artifact of being at the edge of the gel when the western transfer took place. It is worth noting that Akt is similarly low here as well, likely for the same reason.
Figure 6 is a western blot demonstrating that azacytidine upregulated eIF4E downstream targets including XIAP, Mdm2 and Mcl-1, all anti-apoptotic genes in KG1 cells. Further, the methyltransferase inhibitor, e.g., azacytidine, leads to Akt activation which is associated with survival signaling, whereas the inhibitor of eIF4E, e.g., ribavirin, reduces Akt activation (via reducing NBS1 see Figure 6 as well as Tan et al., (2008), "Ribavirin targets elF4E dependent Akt survival signaling". Biochemical and Biophysical Research Communications, Volume 375, Issue 3, 24 October 2008, Pages 341-345) alone or when in combination with the methyltransferase inhibitor, e.g., azacytidine. The inhibitor of eIF4E, e.g., ribavirin, reduces the levels of these targets below that of the methyltransferase inhibitor, e.g. azacytidine alone, suggesting that the inhibitor of eIF4E, e.g. ribavirin, cooperates with the methyltransferase inhibitor, e.g., azacytidine, physiologically by inhibiting eIF4E activity (which azacytidine apparently stimulates in these cells, likely due to some compensatory survival mechanism). Also shown on these western blots are caspase 3 and LC3B (shown as LC3) markers for apoptosis and autophagy respectively. The increase in the lower molecular weight LC3B band indicates increased autophagy and the increase in the cleaved caspase 3 bands (at 19 kDa, 17 kDa and 16 kDa) indicates increased apoptosis. It also appears that the methyltransferase inhibitor, e.g., azacytidine, increases the total amount of LC3B protein relative to untreated or control cells which also may suggest increased cell death. Increased LC3B is present in methyltransferase inhibitor, e.g., azacytidine, alone treated cells but the composition according the present invention, for example a methyltransferase inhibitor with an inhibitor of eIF4E, e.g., azacytidine with ribavirin, combination treated cells have much more cleaved LC3B than any other treatment. Further, both ribavirin and AZA treatment alone and together can lead to cell cycle arrest. Thus, the combination of therapeutic agents according to the present invention preferably reduces cancer cells by potentiating cell death and impeding proliferation.

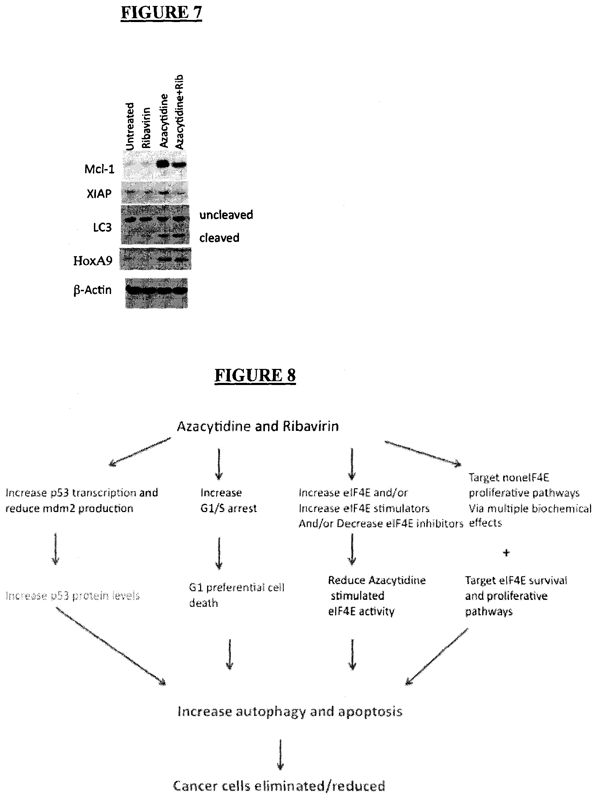

Figure 7 is directed to a western blot demonstrating that methyltransferase inhibitor (e.g., azacytidine) upregulated eIF4E downstream targets and the effects of the inhibitor of eIF4E (e.g., ribavirin), alone or in combination, on eIF4E targets, markers of apoptosis and markers of autophagy in THP-1 cells. The increase in XIAP is not as striking in THP-1 cells as in KG1-a cells (see figure 7) but is still substantial relative to any other treatment. In these cells, Mcl-1 is also upregulated by the methyltransferase inhibitor (e.g., azacytidine) as in KG-1a cells. In both cases, the combination of these drugs leads to increased cell death. The increase in the lower molecular weight LC3 band (and thus cleavage of LC3) indicates increased autophagy. Cleavage is more pronounced in the azacytidine/ribavirin combination treated cells than in either alone. THP1 cells contain much more LC3 than KG1-a cells, and that is why LC3 is detectable in the untreated controls. It also appears that the methyltransferase inhibitor (e.g., azacytidine) increases the total amount of LC3 protein relative to untreated cells which indicates increased cell death.
Figure 8 is a summary description demonstrating some of the pertinent inhibitors of eIF4E (e.g., ribavirin) and methyltransferase inhibitors (e.g., azacytidine) activities. This figure is not a substitute for the information in the present application, but rather a graphical description of some of the more important points.

### DETAILED DESCRIPTION

### 1. Definitions

By "prodrug" is meant to indicate a compound that may be converted under physiological conditions or by solvolysis to a biologically active compound of the invention. Thus, the term "prodrug" refers to a metabolic precursor of a compound of the invention that is pharmaceutically acceptable. A prodrug may be inactive when administered to a subject in need thereof, but is converted in vivo to an active compound of the invention. Prodrugs are typically rapidly transformed in vivo to yield the parent compound of the invention, for example, by hydrolysis in blood. The prodrug compound often offers advantages of solubility, tissue compatibility or delayed release in a mammalian organism (see, for example, Bundgard, H., Design of Prodrugs (1985), pp. 7-9, 21-24 (Elsevier, Amsterdam).

The abbreviation "eIF4E" stands for eukaryotic translation initiation factor 4E, which is a protein which in humans is encoded by the *eIF4E* gene.

By an "inhibitor of eIF4E" is meant any compound that inhibits the biochemical activity of eIF4E including its role in mRNA translation and mRNA export or elF4E levels (RNA or protein). Examples of inhibitors of eIF4E include, for example: ribavirin (1-β,D-ribofuranosyl-1*H*-1,2,4-thiazole-3-carboxamide) and its derivatives. Desirably, an "inhibitor of eIF4E" results in a reduction in cancer or dissemination of, for example, at least 10%, 20%, 30%, or 50% of cancer cells. In more desirable embodiments, an "inhibitor of eIF4E" reduces replication or dissemination, for example, by at least 70%, 80%, 90%, 95%, or even 99%, of cancer cells.

"Pharmaceutically acceptable salt" and "salts thereof" in the compounds of the present invention refers to acid addition salts and base addition salts.

By "methyltransferase inhibitor" or "DNA methyltransferase inhibitor" is meant compounds which inhibit the methylation of the DNA base cytosine at the C-5 position of that base by the DNA methyltransferase enzyme. Examples of methyltransferase inhibitors include those compounds which can inhibit any type of methyltransferase either by binding to it directly or by incorporating into RNA or DNA leading to deficient enzymatic activity. Also included into this definition is any compound that disrupts the proper processing of rRNA, tRNA or mRNA via its incorporation. Specific DNA methyltransferase inhibitors include those cited in Goffin et al., "DNA methyltransferase inhibitors-state of the art", Ann Oncol (2002) 13 (11): 1699-1716. doi: 10.1093/annonc/mdf314, and more particularly, for example: 5-Aza-CdR (5-Aza-2-deoxycytidine, also referred to as decitabine), 5-Aza-CR (5-azacytidine) and zebularine®, amongst other methyltransferase inhibitors known to a person skilled in the art.

The term "low dose" is meant an amount of methyltransferase inhibitor sufficient for repressing the tumorgenicity of cells.

By a "pharmaceutically acceptable carrier" is meant a non-toxic solid, semisolid or liquid filler, diluent, encapsulating material or formulation auxiliary of any type. The use of pharmaceutically acceptable carriers is well known in the art. The type of pharmaceutically acceptable carrier used will depend upon the method of administration of the agent and dosage required. The method and dosage are expected to be determined according to standard procedures for the administration of the composition according the present invention.

By "injectable" is meant a composition or formulation that is suitable for placing into a syringe and injecting into the mammalian body. The composition and vehicle are tissue-compatible and should not rely on constituents that are likely to elicit an allergic response. Injectable compositions can be injected into the mammalian body without causing adverse effects due to the presence of solid materials in the composition. Solid materials include, but are not limited to, particles, crystals, a gummy mass, and a gel.

The injectable compositions can be injected intra-muscularly, intra-venously, subcutaneously, intra-dermally, intra-peritoneally, or parenterally, or other suitable modes of injection into the mammalian body.

The terms "cancer", "cancerous" or "neoplasm" or "neoplastic cells" encompass neoplasms, cancers, or neoplastic cells located at the original site of proliferation ("primary tumor or cancer") and their invasion of other tissues, or organs beyond the primary site ("metastasis"). They also refer to or describe the physiological condition in mammals in which a population of cells is characterized by unregulated cell growth. Examples of cancer include and are not limited to: leukemia, acute myeloid leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia, polycythemia vera, lymphoma, Hodgkin's disease, non-Hodgkin's disease lymphoma, Waldenstrom's macroglobulinemia, heavy chain disease, fibrosarcoma, myxosarcoma, liposarcoma, chondrosarcoma, osteogenic sarcoma, chordoma, angiosarcoma, endotheliosarcoma, lyinphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, retinoblastoma, lung cancer, squamous cell carcinoma, adenocarinoma, large cell carcinoma, colorectal cancer, ovarian cancer, ovarian adenocarcinoma, prostate cancer, myelodysplastic syndromes (MDS) and multiple myeloma.

The term "neoplasm" or "neoplastic" also means a cell or tissue exhibiting abnormal growth, including hyperproliferation or uncontrolled cell growth, that may be benign or cancerous. The development from a normal cell to a cell exhibiting a neoplastic phenotype is a multi-step process. Cells developing a neoplastic phenotype or designated as of a cancerous cell type generally exhibit an alteration of the normal cell cycle and altered apoptotic response. Generally the changes that a cell undergoes in developing to a tumor cell may be monitored at the cellular or DNA level. Therefore, the terms "preneoplasm" or "preneoplastic" phenotype are construed for the purposes of the present invention to refer to a cell or tissue which exhibits changes at the DNA or cellular level that evidence the ultimate progression of the cell or tissue to a neoplastic or cancerous phenotype. Preneoplastic conditions do not show evidence of microinvasion or other hallmarks of cancer behavior. As with the development to neoplasia, preneoplastic cells may exhibit progression through multiple steps. Although a preneoplastic cell may progress to a neoplastic stage, they may remain stable for an extended period of time and may even regress. The development of preneoplasia is often associated with enviromental factors. Examples of preneoplastic conditions in noninvasive bladder cancer include diffuse cellular atypia of the urothelium.

The term "proliferative disorder" refers to disorders that are associated with some degree of abnormal cell proliferation.

The term "precancerous" refers to cells or tissues having characteristics relating to changes that may lead to malignancy or cancer. Examples include adenomatous growths in colon, ovary, breast, tissues, or conditions, for example, dysplastic nevus syndrome, a precursor to malignant melanoma of the skin. Examples also include, abnormal neoplastic, in addition to dysplastic nevus syndromes, polyposis syndromes, prostatic dysplasia, and other such neoplasms, whether the precancerous lesions are clinically identifiable or not. A "precancerous lesion(s)" may refer to an epithelial precancerous lesion, which is a skin lesion that has a propensity to develop into a cancerous condition. Epithelial precancerous skin lesions also arise from other proliferative skin disorders such as hemangiomas, keloids, eczema and papilloma virus infections producing verruca vulbaris, verruca plantaris and verruca planar. The symptoms of the epithelial precancerous lesions include skin-colored or red-brown macule or papule with dry adherent scales. Actinic keratosis is the most common epithelial precancerous lesion among fair skinned individuals. It is usually present as lesions on the skin which may or may not be visually detectable. The size and shape of the lesions varies. It is a photosensitive disorder and may be aggravated by exposure to sunlight. Bowenoid actinic keratosis is another form of an epithelial precancerous lesion. In some cases, the lesions may develop into an invasive form of squamous cell carcinoma and may pose a significant threat of metastasis. Other types of epithelial precancerous lesions include hypertrophic actinic keratosis, arsenical keratosis, hydrocarbon keratosis, thermal keratosis, radiation keratosis, viral keratosis, Bowen's disease, erythroplaquia of queyrat, oral erythroplaquia, leukoplakia, and intraepidermal epithelialoma.

"Metastasis" as used herein refers to the process by which a cancer spreads or transfers from the site of origin to other regions of the body with the development of a similar cancerous lesion at the new location. A "metastatic" or "metastasizing" cell is one that loses adhesive contacts with neighboring cells and migrates via the bloodstream or lymph from the primary site of disease to invade neighboring body structures.

"Infiltration" as used herein refers to the process by which leukemia is spread or transfers from the site of origin to other regions of the body with the development of a similar leukemic lesion at the new location. Sites of infiltration can include the skin, CNS, etc. An "infiltrating" cell is one that leaves the bone marrow or peripheral blood and invades other parts of the body.

By "inhibits the growth of a neoplasm" is meant measurably slows, stops, or reverses the growth rate of the neoplasm or neoplastic cells in vitro or in vivo. Desirably, a slowing of the growth rate is by at least 20%, 30%, 50%, or even 70%, over a period of treatment of six month is achieved as determined using a suitable assay for determination of cell growth rates (e.g., a cell growth assay described herein). Typically, a reversal of growth rate is accomplished by initiating or accelerating necrotic or apoptotic mechanisms of cell death in the neoplastic cells, resulting in shrinkage of the neoplasm.

By "an effective amount", "a neoplasm treating amount", "a preneoplasm treating amount", "a proliferative treating amount" or by "a precancerous lesion treating amount" is meant the amount of a compound or a combination of compounds required to treat or prevent a disease in a clinically relevant manner. An effective amount may be inhibitory, prophylactic and/or therapeutic. Compounds according to the present invention are particularly useful for providing favorable change in the disease or condition treated, whether that change is a remission, a decrease in growth or size of cancer or a tumor or other effect of the condition or disease to be treated, a favorable physiological result or a reduction in symptomology associated with the disease or condition treated. An effective amount or a treating amount of a compound varies depending upon the disease being treated, the manner of administration, and the age, body weight, and general health of the patient. Ultimately, the prescribers will decide the appropriate amount and dosage regimen according to good medical practice.

The expressions "therapeutic effective amount" or "therapeutically effective amount" are used throughout the description to describe concentrations or amounts of compounds according to the present invention which are therapeutically effective in treating neoplasms (i.e. tumors, cancers, etc.), preneoplasms, proliferative disorders, and/or precancerous lesions or the various conditions or disease states including hyperproliferative cell growth, psoriasis and related conditions, as well as arthritis and chronic inflammatory diseases, including rheumatoid arthritis and osteoarthritis, among others.

The expression "preventing effective amount" is used throughout the specification to describe concentrations or amounts of compounds according to the present invention which are prophylactically effective in preventing, reducing the likelihood of contracting or delaying the onset of one or more of the disease states according to the present invention. Within the context of the present invention, a preventing effective amount is an amount, for example, which may reduce the likelihood that a precancerous lesion may become a malignant tumor or that a non-malignant tumor will become malignant. This term is subsumed under the term "effective amount". Certain compounds according to the present invention are particularly useful as prophylactic agents because of the reduced toxicity these compounds exhibit to non-tumorigenic and/or non-cancerous cells.

The term "administration" or "administering" refers to a method of giving a composition of the invention to a patient, by a route such as inhalation, ocular administration, nasal instillation, parenteral administration, dermal administration, transdermal administration, buccal administration, rectal administration, sublingual administration, perilingual administration, nasal administration, topical administration and oral administration. Parenteral administration includes intrathecal, intraarticular, intratumoral, intravenous, intraperitoneal, subcutaneous, and intramuscular administration. The optimal method of administration of a drug or drug combination to treat a particular disease can vary depending on various factors, e.g., the oral bioavailability of the drug(s), the anatomical location of the disease tissue, and the severity of disease.

The term "western blot" refers to the analysis of protein(s) (or polypeptides) immobilized onto a support such as nitrocellulose or a membrane. The proteins are run on polyacrylamide gels to separate the proteins, followed by transfer of the protein from the gel to a solid support, such as nitrocellulose, polyvinylidene fluoride (PVDF) or a similar membrane. The immobilized proteins are then exposed to antibodies with reactivity against an antigen of interest. The binding of the antibodies can be detected by various methods, including the use of radio-labeled antibodies or chemiluminescence.

### 2. The Preferred combination: Azacytidine and Ribavirin

Ribavirin was used extensively as a broad-spectrum anti-viral therapy. Thanks to the support of The Leukemia and Lymphoma Society (USA), the Applicant tested the efficacy of ribavirin treatment in patients in a Canada-wide clinical trial, and observed striking improvement with the patients, however, all eventually developed resistance to ribavirin (Assouline et al., Blood, 2009).

To overcome this resistance problem of ribavirin, and according to the present invention, there is provided a novel pharmaceutical composition for treating a neoplasm, a preneoplasm, a proliferative disorder, and a precancerous lesion. The pharmaceutical composition according to the present invention is made up of ribavirin, a methyltransferase inhibitor, and a pharmaceutically acceptable carrier, wherein the methyltransferase inhibitor is azacytidine. This novel combinatory drug therapy is useful due the cooperation between the methyltransferase inhibitor (azacytidine) and the inhibitor of eIF4E (ribavirin). To better understand this dynamic interaction between these two inhibitors on cancerous or precancerous cells, a review is provided below.

### 3. Cooperation between the inhibitor of eIF4E and methyltransferase inhibitors

It has been noted that a key pathway disrupted in many cancers, including leukemias, is the methylation of DNA and RNA. An inhibitor of this methylation process is preferably azacytidine (also known as Vidaza®). Given that azacytidine can inhibit RNA and DNA methyltransferases, it is likely that azacytidine could potentiate ribavirin activity by inhibiting or reducing the formation of methyl 7-guanosine caps on the 5' end of normal mRNAs. In addition, or alternatively to this, azacytidine is known to be incorporated into cellular RNAs and for this reason could also reduce translation of RNAs that were either eIF4E sensitive or insensitive. Several other modes of collaboration are described below. Thus, in a preferred embodiment, azacytidine could potentiate the activities of ribavirin for the following reasons:
1. that azacytidine is cytotoxic; whereas ribavirin is cytostatic;
2. that ribavirin inhibits eIF4E dependent oncogenic transformation (leaving other pathways alone) whereas azacytidine through its RNA and DNA methylation and/or incorporation activities can modulate different distinct pathways that may also be supporting oncogenic transformation in these cells;
3. that azacytidine can help ribavirin better inhibit its targets by either blocking proper 5' cap formation on these mRNAs and/or by incorporating into cellular RNAs and further inhibiting their translation (or export);
4. that azacytidine may block the translation or export of mRNAs that are not particularly eIF4E sensitive, thereby increasing the population of different RNAs (and thus pathways) that are targeted and thereby improving physiological response;
5. that azacytidine promotes production of p53; whereas ribavirin reduces mdm2 (so increases p53); the combination could thus synergize on this pro-apoptotic and pro-autophagic pathway;
6. there is evidence that a key inhibitor of eIF4E, PML, binds to the methyltransferase DNMT1 and modulates PML activity in PML nuclear bodies and visa versa; the modulation of DNMT1 activity may augment eIF4E activity via PML;
7. that azacytidine modulates the transcription of eIF4E regulatory proteins, thereby modulating the eIF4E function;
8. that ribavirin modulates eIF4E dependent mRNA translation and mRNA export and that azacytidine inhibits translation through multiple mechanisms inducing degradation of 45S pre-rRNA (Reichman et al., 1973 BBA), interfering with tRNA metabolism (Lu et al., 1976, BBRC; Lu and Randerrath Cancer Research 1979) and being misincorporated into mRNA, tRNA and rRNA (Hollenbach et al., PLOS One 2010, and references therein). In fact, 65-85% of cellular azacytidine is found incorporated into RNA (Hollenbach et al, PLOS One 2010). Thus, ribavirin and azacytidine likely effect distinct, nonoverlapping targets allowing the combination of the present invention to more efficiently modulate gene expression leading to reduced growth and increased cell death of cancer cells;
9. that azacytidine cooperates with ribavirin by targeting the mRNA metabolism of genes involved in the regulation of eIF4E directly, of the regulation of the regulators;
10. Azacytdine induced apoptosis is preferential to G1 phase cells (Murakami 1995 Cancer Res; and Gorzyca, 1993 Cancer Res). Ribavirin induces a G1/S arrest in many cell types (Kentsis et al., 2004). Thus, ribavirin can potentiate this activity of azacytidine in this way; and
11. that ribavirin potentiates azacytidine mediated apoptosis and autophagy meaning that these drugs can cooperate to promote multiple types of cell death simultaneously in the same cell population.

The Applicant has examined the effects of the combination of an inhibitor of eIF4E, preferably ribavirin, with a methyltransferase inhibitor, e.g., azacytidine. Azacytidine is a nucleotide analogue that is misincorporated into DNA during replication and into RNA during transcription leading to demethylation. Further, azacytidine is misincorporated into tRNA and rRNA as well as mRNA. Thus, it leads to gross changes in cellular ribosomes, incorrectly modified tRNAs and thus generally can effect RNA processing and translation. The Applicant has examined whether the inhibitor of eIF4E (e.g., ribavirin) and the methyltransferase inhibitor (e.g., azacytidine) cooperated in primary AML specimens (purchased from the BCLQ). These studies looked at colony growth in methyl cellulose. The Applicant has observed that azacytidine alone leads to a modest reduction in colony number, with around 70% of the colonies observed for untreated control, as observed in Figure 1. It is worth noting that, in this study, 3 µM of azacytidine was preferably used, which is well within the 3 to 11 µM range of azacytidine used in patients.

It has also been demonstrated that the preferred combination of methyltransferase inhibitor (e.g., azacytidine) with the inhibitor of eIF4E (e.g., ribavirin) led to a striking reduction in colony number to about 10-30% of the untreated cells. Relative to azacytidine alone, responses were two to ten-fold better than azacytidine alone. Further, an additional 10 to 34% inhibition was observed versus cells treated with ribavirin alone. The best responders to the combination therapy according to the present invention were AML-M4 FLT3 wildtype patients with only 7% of colonies relative to untreated controls. In AML M5 specimens that carried the Flt3 mutation, the methyltransferase inhibitor (e.g., azacytidine) alone had approximately the same effect as in Flt3 wildtype specimens. Further, the effects of the inhibitor of eIF4E (e.g., ribavirin) alone, or the methyltransferase inhibitor (e.g., azacytidine) alone were independent of Flt3 status. Thus, the combination of the eIF4E inhibitor (e.g., ribavirin) with the methyltransferase inhibitor (e.g., azacytidine) acts better than either drug alone to inhibit colony growth. The most sensitive was the M4 AML with wildtype Flt3, as demonstrated in Figure 1 of this document.

The Applicant has also examined the effects of ribavirin and azacytidine (alone and in combination) in an M1 AML specimen with elevated eIF4E (see Figures 1 and 4). The Applicant's previous studies indicated that M1 and M2 AML only rarely had elevated eIF4E protein levels (a frequency of about 10%) (Culjkovic-Kraljacic B, Borden KL, "Rivabirin as an anti-cancer therapy: actute myeloid leukemia and beyond?", Leuk Lymphoma, 2010 Oct; Vol. 51(10); pp. 1805-15). In the present case, the Applicant examined whether these cells would still be sensitive to the inhibitor of eIF4E, e.g., ribavirin, and the combination, using methyl-cellulose colony assays. The Applicant found that M1-high4E responded to the inhibitor of eIF4E, e.g., ribavirin, very similarly to M4 and M5 AML (with around 40% of colonies versus untreated controls, i.e. a 60% reduction) (Figure 1). In contrast, M1 and M2 AML specimens (with normal eIF4E levels) only modestly respond to the inhibitor of elF4E (e.g., ribavirin) with a 70-80% the colonies versus untreated cells (only a 25% drop or so). It can be noted that M1-high4E specimen with a methyltransferase inhibitor (e.g., azacytidine) alone was about 80% of the untreated control, but the combination with an inhibitor of eIF4E (e.g., ribavirin) led to a dramatic reduction to 28% of the untreated control. When the inhibitor of eIF4E, i.e. ribavrin, was used alone, the reduction was 43%, and thus the cells responded to the combination according to the present invention better than with either drug alone. Thus high eIF4E, not lineage, predicts response to the inhibitor of eIF4E, e.g., ribavirin, and to the combination of the present invention, preferably ribavirin with azacytidine.

From Figures 6 and 7, it can be noted that the inhibitor of eIF4E, more particularly ribavirin, inhibits eIF4E activity in azacytidine treated samples, suggesting that targeting the eIF4E pathways is effective.

It is worth noting that the capping of mRNAs requires a specific methylation step of the 5' cap by guanine-7-methyl transferase. This enzyme is S-adenosyl-methionine (SAM) dependent. The methyltransferase inhibitor (e.g. azacytidine) can also reverse SAM dependent methylation in the case of other enzymes. Further, the effects of the methyltransferase inhibitor (e.g., azacytidine) on capping could also be indirect, in that it can effect the expression of the enzyme or cofactors. For instance, azacytidine interferes with the methylation of tRNA, mRNA and rRNA.

The Applicant also observed an interesting relationship between the preferred combination of azacytidine and ribavirin during experimentation. Indeed, it appears that the methyltransferase inhibitor (e.g., azacytidine), alone, seemed to stimulate eIF4E activity in at least some cell types and perhaps in cell type specific manner. For instance, azacytidine elevated XIAP, mdm2 and Mcl-1 in KG1-a and THP-1 cells (all of which are eIF4E targets; see Figures 6 and 7). It was also observed that the methyltransferase inhibitor (e.g., azacytidine) can stimulate the production of eIF4E itself (Figure 6), which would be consistent with an elevation of the downstream targets of eIF4E alone. It is also thought that it lowers some elF4E inhibitors as well as promotes activators through methylation processes. The Applicant has also discovered that eIF4E is a direct transcriptional target of NFKB. One of the key subunits of NFkappaB is p65, and the Applicant has noted that levels of this subunit are elevated by azacytidine treatment (Figure 6).

The Applicant noted, in western blot analyses of AML tissue culture cell lines (see Figure 5, 6 and 7), that ribavirin knocked down eIF4E targets which were not targeted with azacytidine alone. The Applicant further noted, rather surprisingly, that eIF4E targets became elevated in cells treated with methyltransferase inhibitors (e.g., azacytidine) alone (XIAP, mdm2 and Mcl-1), but in the combination with an inhibitor of eIF4E (e.g., ribavirin) could substantially reduce the levels of these targets. Thus, a failure in physiological response to a methyltransferase inhibitor (e.g., azacytidine) alone may be that it activates the elF4E pathway (or a subset of the eIF4E pathway). Such activation may be an indirect result of its affects on RNA metabolism and/or its effects on transcription through DNA methylation. However, it is clear that the use of an inhibitor of eIF4E (e.g., ribavirin) in combination with a methyltransferase inhibitor (e.g., azacytidine) can temper this affect leading to better physiological responses.

Alternatively, the methyltransferase inhibitor, e.g., azacytidine, may also start a compensatory mechanism wherein the cells try to evade apoptosis by elevating eIF4E level and/or activity (see Figure 6), and in this case, the inhibitor of eIF4E, preferably ribavirin, quenches that by inhibiting eIF4E activity; thereby enabling a cooperative effect between the two drugs.

The Applicant studied in depth the preferred combination of ribavirin and azacytidine, since azacytidine promotes production of p53, whereas ribavirin reduces mdm2 (Figure 5) and increased p53 (see Figure 8). The preferred combination could therefore synergize this pro-apoptotic and/or pro-autophagic cell death pathway. There is also some evidence that a key inhibitor of eIF4E, PML, binds to the methyltransferase DNMT1 (in PML nuclear bodies). Thus, a methyltransferase inhibitor (e.g., azacytidine) could be modulating/promoting PML activity by inhibiting DNMT1. It is possible that the methyltransferase inhibitor (e.g., azacytidine) modulates the transcription/translation/mRNA export/RNA processing of eIF4E regulatory proteins thereby modulating eIF4E function. At first, the Applicant thought that the methyltransferase inhibitor (e.g., azacytidine) would be promoting the production of an inhibitor. However, given the data (see Figures 6 and 7) it might be that the methyltransferase inhibitor (e.g., azacytidine) is actually promoting the production of a stimulator of eIF4E, such as HoxA9. HoxA9 is a stimulator shown in Topisirovic 2005 MCB.

In this connection, the Applicant has also observed that the methyltransferase inhibitor, e.g., azacytidine, elevates HoxA9 protein levels (see Figure 7). Ribavirin does not reverse this, but can inhibit eIF4E activity by directly acting on the eIF4E protein thereby resulting in decreased eIF4E activity, as the Applicant has shown previously. In the scientific literature, there is some evidence that azacytidine promotes demethylation of the HoxA9 gene and thus this seems a likely mechanism (Milne 2002 Molecular Cell for Hox genes being targets of methyltransferases; Thea Tlsty Genetic and Epigenetic changes in Early Carcinogenesis, 20th Aspen Conference http://www.ncbi.nlm.nih.gov/pmc/articles/PMC2610844/). Thus, the methyltransferase inhibitor, e.g., azacytidine, can stimulate eIF4E activity by de-repressing the transcription of a stimulator of eIF4E activity, HoxA9. Also, the inhibitor of eIF4E, e.g., ribavirin, can promote a G1/S cell cycle arrest and azacytidine mediated cell death is preferential to G1, thus this might also explain how the inhibitor of eIF4E, e.g., ribavirin, can potentiate the activity of azacytidine.

### 3. Other agents that May be Used in Conjunction with the Combination of inhibitor of eIF4E and methyltransferase inhibitor.

It is not uncommon in cancer treatments to have mixtures of three or more drugs. In this connection, other agents may be used in conjunction with the combination of inhibitor of eIF4E and methyltransferase inhibitor. For example, the composition according to present invention may further comprise at least one other pharmaceutically active substance. Other pharmaceutically active substance(s), include, and are not limited to: topoisomerase inhibitors, NFκB inhibitors, anthracyclines and cisplatin. Suitable topoisomerase inhibitors may include, and are not limited to: etoposide, topotecan, camptothecan, hycaptamine, irinotecan, rubitecan, 6-ethoxypropionyl-3',4'-O-exo-benzylidene-chartreusin, 9-methoxy-N,N-dimethyl-5-nitropyrazolo[3,4,5-kl]acridine-2-(6H) propanamine, 1-amino-9-ethyl-5-fluoro-2,3-dihydro-9-hydroxy-4-methyl-1H,12H-benzo[de]p- yrano[3',4':b,7]-indolizino[1,2b]quinoline-10,13(9H, 15H)dione, lurtotecan, 7-[2-(N-isopropylamino)ethyl]-(20S)camptothecin, BNP1350, BNPI1100, BN80915, BN80942, etoposide phosphate, teniposide, sobuzoxane, 2'-dimethylamino-2'-deoxy-etoposide, GL331, N-[2-(dimethylamino)ethyl]-9-hydroxy-5,6-dimethyl-6H-pyrido[4,3-b]carbazo- le-1-carboxamide, asulacrine, (5a, 5aB, 8aa,9b)-9-[2-[N-[2-(dimethylamino)ethyl]-N-methylamino]ethyl]-5-[4-hydrox- y-3,5-dimethoxyphenyl]-5,5a,6,8,8a,9-hexohydrofuro(3',4':6,7)naphtho(2,3-d- )-1,3-dioxol-6-one, 2,3-(methylenedioxy)-5-methyl-7-hydroxy-8-methoxybenzo[c]-phenanthradiniu- m, 6,9-bis[(2-aminoethyl)amino]benzo[g]isoquinoline-5,10-dione, 5-(3-aminopropylamino)-7,10-dihydroxy-2-(2-hydroxyethylaminomethyl)-6H-py- razolo[4,5,1-de]acridin-6-one, N-[1-[2(diethylamino)ethylamino]-7-methoxy-9-oxo-9H-thioxanthene-4-ylmeth- yl]formamide, N-(2-(dimethylamino)ethyl)acridine-4-carboxamide, 6-[[2-(dimethylamino)ethyl]amino]-3-hydroxy-7H-indeno[2,1-c]quinolin-7-one, dimesna, and their derivatives.

Suitable NFκB inhibitor(s) may include, and are not limited to natural antioxidants and synthetic NFκB inhibitors. For example, natural antioxidants are selected from the group consisting of: isoflavone genistein, indole-3-carbinol (13C), 3,3'-diindolylmethane (DIM), curcumin, (-)-epigallocatechin-3-gallate (EGCG), resveratrol, lycopene, vitamin E and vitamin C, alone or in combination. Synthetic NFκB inhibitors are selected from the group consisting of: dehydroxymethylpoxyquinomicin, cyclooxygenase-2-inhibitors, parthenolide, sulfasalazine, proteasome inhibitors, (E)-3-(4-Methylphenylsulfonyl)-2-propenenitrile (BAY 11-7082), N-[3,5-Bis(trifluoromethyl)phenyl]-5-chloro-2-hydroxybe nzamide (IMD-0354) and SAHA, alone or in combination. Part of the class of proteasome inhibitors, include for example: PS-341 and MG-132. A particular example of PS-341 is bortezomib (commercially available as velcade®). Suitable anthracyclines include and are not limited to daunorubicin, doxorubicin, epirubicin and idarubicin, alone or in combination.

In a preferred embodiment, drugs that inhibit NFκB would be of interest, as they may exhibit particular pK properties. Such drugs would include, but are not limited to, parthenolide and its derivatives. These compounds (or drugs) would allow for the inhibition eIF4E production that azacytidine causes by elevation of p65. Other drugs include:
- velcade® which inhibits many things including NFkB;
- sorafenib because so many of the patients harbour Flt3 mutations;
- cisplatin which cooperates with antisense eIF4E; and
- Topoisomerase inhibitors, including those mentioned hereinabove.

### 4. Indications for Treatment

The compositions of the present invention are for the treatment of conditions which involve undesirable or uncontrolled cell proliferation, namely neoplasms (i.e. cancer), pre-neoplasms, proliferative disorders and precancerous lesions.

In a preferred embodiment, the neoplasm is cancer. Preferably, the cancer is selected from the group consisting of leukemia, acute myeloid leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia, polycythemia vera, lymphoma, Hodgkin's disease, non-Hodgkin's disease lymphoma, Waldenstrom's macroglobulinemia, heavy chain disease, fibrosarcoma, myxosarcoma, liposarcoma, chondrosarcoma, osteogenic sarcoma, chordoma, angiosarcoma, endotheliosarcoma, lyinphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, retinoblastoma, lung cancer, squamous cell carcinoma, adenocarinoma, large cell carcinoma, colorectal cancer, ovarian cancer, ovarian adenocarcinoma, prostate cancer, myelodysplastic syndromes, and multiple myeloma.

In a preferred embodiment, the acute myeloid leukemia is acute myeloid leukemia M4 or acute myeloid leukemia M5 or another AML subtype characterized by atypical elevation of eIF4E.

Other types of cancers which could potentially be treated include, but are not limited to, leukemia, acute myeloid leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia, polycythemia vera, lymphoma, Hodgkin's disease, non-Hodgkin's disease lymphoma, Waldenstrom's macroglobulinemia, heavy chain disease, fibrosarcoma, myxosarcoma, liposarcoma, chondrosarcoma, osteogenic sarcoma, chordoma, angiosarcoma, endotheliosarcoma, lyinphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, retinoblastoma, lung cancer, squamous cell carcinoma, adenocarinoma, large cell carcinoma, colorectal cancer, ovarian cancer, ovarian adenocarcinoma, prostate cancer, myelodysplastic syndromes and multiple myeloma.

Generally, cells in a benign tumor retain their differentiated features and do not divide in a completely uncontrolled manner. A benign tumor is usually localized and nonmetastatic. Specific types of benign tumors that can be treated using the present invention include hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas.

In a malignant tumor cells become undifferentiated, do not respond to the body's growth control signals, and multiply in an uncontrolled manner. The malignant tumor is invasive and capable of spreading to distant sites (metastasizing). Malignant tumors are generally divided into two categories: primary and secondary. Primary tumors arise directly from the tissue in which they are found. A secondary tumor, or metastasis, or even infiltrating leukemia cells, is a tumor which is originated elsewhere in the body but has now spread to a distant organ. The common routes for metastasis are direct growth into adjacent structures, spread through the vascular or lymphatic systems, and tracking along tissue planes and body spaces (peritoneal fluid, cerebrospinal fluid, etc.). Malignant leukemia cells can infiltrate into other tissues.

Specific types of cancers or malignant tumors, either primary or secondary, that can potentially be treated using the present invention include leukemia, breast cancer, skin cancer, bone cancer, prostate cancer, liver cancer, lung cancer, brain cancer, cancer of the larynx, gall bladder, pancreas, rectum, parathyroid, thyroid, adrenal, neural tissue, head and neck, colon, stomach, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumor, small-cell lung tumor, gallstones, islet cell tumor, primary brain tumor, acute and chronic lymphocytic and granulocytic tumors, hairy-cell tumor, adenoma, hyperplasia, medullary carcinoma, pheochromocytoma, mucosal neuronms, intestinal ganglloneuromas, hyperplastic comeal nerve tumor, marfanoid habitus tumor, Wilm's tumor, seminoma, ovarian tumor, leiomyomater tumor, cervical dysplasia and in situ carcinoma, neuroblastoma, retinoblastoma, soft tissue sarcoma, malignant carcinoid, topical skin lesion, mycosis fungoide, rhabdomyosarcoma, Kaposi's sarcoma, osteogenic and other sarcoma, malignant hypercalcemia, renal cell tumor, polycythermia vera, adenocarcinoma, glioblastoma multiforma, leukemias, lymphomas, malignant melanomas, epidermoid carcinomas, and other carcinomas and sarcomas.

Hematologic disorders include abnormal growth of blood cells, which can lead to dysplastic changes in blood cells and hematologic malignancies such as various leukemias. Examples of hematologic disorders include but are not limited to acute myeloid leukemia, acute promyelocytic leukemia, acute lymphoblastic leukemia, chronic myelogenous leukemia, the myelodysplastic syndromes (MDS), multiple myeloma and sickle cell anemia.

Acute myeloid leukemia (AML) is the most common type of acute leukemia that occurs in adults. Several inherited genetic disorders and immunodeficiency states are associated with an increased risk of AML. These include disorders with defects in DNA stability, leading to random chormosomal breakage, such as Bloom's syndrome, Fanconi's anemia, Li-Fraumeni kindreds, ataxia-telangiectasia, and X-linked agammaglobulinemia.

Acute promyelocytic leukemia (APML) represents a distinct subgroup of AML. This subtype is characterized by promyelocytic blasts containing the 15;17 chromosomal translocation. This translocation leads to the generation of the fusion transcript comprised of the retinoic acid receptor and a sequence PML.

Acute lymphoblastic leukemia (ALL) is a heterogenerous disease with distinct clinical features displayed by various subtypes. Reoccurring cytogenetic abnormalities have been demonstrated in ALL. The most common cytogenetic abnormality is the 9;22 chromosomal translocation (known as the Philadelphia chromosome). The resultant Philadelphia chromosome represents poor prognosis of the patient.

Chronic myelogenous leukemia (CML) is a clonal myeloproliferative disorder of a pluripotent stem cell. CML is characterized by a specific chromosomal abnormality involving the translocation of chromosomes 9 and 22, creating the Philadelphia chromosome. Ionizing radiation is associated with the development of CML. Chronic phase CML is often successfully treated with Gleevec®. However, when this converts to blast crisis CML, Gleevec® is not effective. Our previous studies indicate that eIF4E levels are elevated in blast crisis CML, but not in the chronic phase. This means that blast crisis CML patients could be candidates for the combination therapy according to the present invention.

The myelodysplastic syndromes (MDS) are heterogeneous clonal hematopoietic stem cell disorders grouped together because of the presence of dysplastic changes in one or more of the hematopoietic lineages including dysplastic changes in the myeloid, erythroid, and megakaryocytic series. These changes result in cytopenias in one or more of the three lineages. Patients afflicted with MDS typically develop complications related to anemia, neutropenia (infections), or thrombocytopenia (bleeding). Generally, from about 10% to about 70% of patients with MDS develop acute leukemia. MDS patients could be candidates for this therapy.

### 5. Routes of Administration and Dosing Regimen

A number of routes of administration and formulations may be used in the combination therapies of the present invention.

The combination of therapeutic agents according to the present invention may be administered in combination with one or more conventional pharmaceutical excipients. Additionally, the compositions may include active agents in addition to the combination of therapeutic agents according to the present invention. These additional active agents may include additional compounds according to the invention, or one or more other pharmaceutically active agents. In preferable embodiments, the compositions according to the present invention will contain the combination of therapeutic agents, in an amount effective to treat an indication of interest.

The compositions according to the present invention may be administered or co-administered orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, or intrathecally. More particularly, other forms of administration include, for example: inhalation, ocular administration, nasal instillation, parenteral administration, dermal administration, transdermal administration, buccal administration, rectal administration, sublingual administration, perilingual administration, nasal administration, topical administration, and oral administration. The compounds of the compositions according to the present invention may also be administered or co-administered, sequentially or not, in immediate release, delayed release or even slow release dosage forms. In a preferred embodiment, the inhibitor of eIF4E and methyltransferase inhibitor, according to the present invention, are administered sequentially or simultaneously.

The compositions according to the present invention may be administered by a variety of routes, and may be administered or coadministered in any conventional dosage form. In a preferred embodiment, the composition, according the present invention, is in a unit dosage form. Co-administration in the context of this invention is defined to mean the administration of more than one therapeutic in the course of a coordinated treatment to achieve an improved clinical outcome. Such co-administration may also be coextensive, that is, occurring during overlapping periods of time. For example, the methyltransferase inhibitor may be administered to a patient before, concomitantly, or after the inhibitor of eIF4E is administered. In a preferred embodiment, the patient may be pretreated with the methyltransferase inhibitor (i.e., azacytidine) and then treated with the inhibitor of eIF4E (i.e., ribavirin).

The amounts of the therapeutic agents present in the compositions of the present invention can vary, according to determinations made by a person skilled in the art, but preferably are in amounts effective to create a cytotoxic or cytostatic effect at the desired site. Preferably, these total amounts are less than the total amount adding up to the maximum tolerated dose for each of the methyltransferase inhibitor and the eIF4E inhibitor, and more preferably less than the total amount added for individual administration of each of these inhibitors. In a preferred embodiment, the amount of therapeutic agent(s), i.e., an inhibitor of eIF4E and/or a methyltransferase inhibitor, are deemed to be in an effective amount for treating the indication, for example: a neoplasm (i.e. cancer or, more particularly, acute myeloid leukemia), a preneoplasm, a proliferative disorder, or a precancerous lesion. Preferably, for the dosage form, appropriate release times can vary, but preferably should last from about 1 hour to about 6 months, and most preferably from about 1 week to about 4 weeks. Formulations including the compositions according to the present invention can vary, as determinable by a person skilled in the art, according to the particular situation, and as generally taught herein.

In a preferred embodiment, the inhibitor of eIF4E and methyltransferase inhibitor are in present in a ratio ranging from about 1:5 to about 5:1, and more preferably in a ratio ranging from about 1:3 to about 3:1. In another preferred embodiment, the inhibitor of eIF4E is administered in an amount between about 500 to about 4400 mg per day, and more preferably, the inhibitor of eIF4E is administered in an amount ranging between about 1000 to about 2800 mg per day, and the methyltransferase inhibitor is administered in an amount between 50 mg/m² to about 150 mg/m², more preferably, at the methyltransferase inhibitor is administered at about 100 mg/m². This administration can preferably last for up to 7 days every 4 weeks, on a repetitious basis if required. Preferably, the methyltransferase inhibitor is administered in an amount sufficient to repress tumorgenicity of cells. More preferably, the methyltransferase inhibitor is administered in a low dose. In this connection, a low dose of methyltransferase inhibitor can preferably range from about 5 to about 15 nM.

Also according to the present invention, after the treatment with the methyltransferase inhibitor and inhibitor of eIF4E, the patient may be further treated with various anticancer agents described above. Owing to the sensitizing effects of the combination therapy on the cells to apoptosis, the dosage of anticancer agents used for the treatment may be lower than that used in a conventional cancer treatment regimen. Thus, a better clinical outcome may be achieved by using the compositions of the present invention.

The following examples are intended to illustrate the invention.

### EXAMPLES AND RESULTS

As aforementioned, the pharmaceutical composition according to the present invention comprises an inhibitor of eIF4E and a methyltransferase inhibitor. In a preferred embodiment, and in a laboratory setting, the Applicant made a composition containing 1 µM of an inhibitor of eIF4E and 3 µM of a methyltransferase inhibitor. More particularly, the Applicant used 1 µM of ribavirin and 3 µM of azacytidine in primary specimens, and even more particularly 10 µM of ribavirin and 3 µM of azacytidine in tissue culture lines.

Thus, in a preferred embodiment the ratio of an inhibitor of eIF4E and methyltransferase inhibitor can range from about 1:5 to about 5:1, and even more particularly, from 1:3 to about 3:1. For patients, they could tolerate at least between about 500 mg/day to about 4400 mg/day of ribavirin and at least between 50 mg/m² to 150 mg/m² of azacytidine. More particularly, a patient could tolerate between about 1000 and 2800 mg/day of ribavirin and up to about 100 mg/m² of azacytidine. In even a more preferred embodiment, the inhibitor of elF4E and the methyltransferase inhibitor is administered at the above noted amounts for up to 7 days every 4 weeks. Put otherwise, a patient, in a preferred embodiment, is provided at least between about 10 µM and about 20 µM of ribavirin and about 3 to about 11 µM azacytidine in the plasma.

The effects of either drug treatment alone or in combination, for AML specimens, is disclosed hereinabove as well as in Figure 1. AML specimens were purchased from the BCLQ. In Figure 1, it is noted that when specimens from different individuals with AML were used to further confirm the results. Here "a" refers to the fact that specimens from 3 different individuals were used and "b" the specimens from 2 different individuals were used. All experiments were repeated at least 5 times with the standard deviation shown. Two different normals were used. Here cells were isolated from either peripheral blood (PBMC) or from early progenitors (CD34+) from healthy volunteers (these were purchased from Stem Cell technologies).

The Applicant has also examined the effects of the combination of an inhibitor of eIF4E, preferably ribavirin with a methyltransferase inhibitor, e.g., azacytidine. Azacytidine is a nucleotide analogue that is misincorporated into DNA during replication and into RNA during transcription leading to demethylation. Further, azacytidine is misincorporated into tRNA and rRNA as well as mRNA. Thus, it leads to gross changes in cellular ribosomes, incorrectly modified tRNAs and thus generally can effect RNA processing and translation. The Applicant has examined whether the inhibitor of eIF4E (e.g., ribavirin) and the methyltransferase inhibitor (e.g., azacytidine) cooperated in primary AML specimens (purchased from the BCLQ). These studies looked at colony growth in methyl-cellulose as the physiological output. The Applicant observed that the methyltransferase inhibitor, e.g., azacytidine, alone leads to a modest reduction in colony number, with around 70% of the colonies observed for untreated control, as observed in Figure 1. Note that in Figure 1, and in a preferred embodiment, 3 µM of azacytidine was used, which is concordant with plasma concentrations of azacytidine found in patients (Marucci G., et al., "Bioavailability of azacitidine subcutaneous versus intravenous in patients with myelodysplastic syndromes" 2005 J Clin Pharmacol, Vol. 45(5): 597-602; Cashen AF., et al. "Pharmacokinetics of decitabine administered as a 3-h infusion to patients with acute myeloid leukemia (AML) or myelodysplastic syndrome (MDS)," 2008, Cancer Chemo Pharm 61(5): 759-66; Blum W. 2008 J Clin Oncol*.*). In a preferred embodiment, ribavirin is used in the range of 10-20 micromolar within the range of clinical use given ribavirin concentrations are typically 10-20 micromolar in plasma from patients (Assouline et al, Blood 2009). 1 µM is used given it is close to the affinity constant for ribavirin for elF4E (Kentsis 2004).

It has also been demonstrated that the preferred combination of methyltransferase inhibitor (e.g., azacytidine) with the inhibitor of eIF4E (e.g., ribavirin) led to a striking reduction to about 10 to 30% compared to the untreated cells. Relative to azacytidine alone, responses were two to ten-fold better than azacytidine alone. Further, an additional 10 to 34% inhibition was observed versus cells treated with ribavirin alone. The best responders to the combination were AML-M4 FLT3 wildtype patients with only 7% of colonies relative to untreated controls. In AML M5 specimens that carried the Flt3 mutation, the methyltransferase inhibitor (e.g., azacytidine) alone had approximately the same effect as in Flt3 wildtype specimens. Further, the effects of the inhibitor of eIF4E (e.g., ribavirin) alone, or the methyltransferase inhibitor (e.g., azacytidine) alone were independent of Flt3 status. Thus, it appears that the combination of the eIF4E inhibitor (e.g., ribavirin) with the methyltransferase inhibitor (e.g., azacytidine) acts better than either drug alone. The most sensitive was the M4 AML with wildtype Flt3, as demonstrated in Figure 1.

The Applicant also examined the effects of the composition according to the present invention, for example ribavirin and azacytidine (alone and in combination), in an M1 AML specimen with elevated eIF4E (M1-high4E). The Applicant's previous studies indicated that M1 and M2 AML only rarely had elevated eIF4E protein levels (a frequency of about 10%). The Applicant examined whether these cells would still be sensitive to ribavirin, and the combination, using methyl-cellulose colony assays. The Applicant found that M1-high4E responded to the inhibitor of eIF4E, e.g., ribavirin, very similarly to M4 and M5 AML (with around 40% of colonies versus untreated controls, so a 60% reduction). In contrast, M1 and M2 AML specimens (with normal eIF4E levels) only modestly respond to the inhibitor of eIF4E (e.g., ribavirin) with a 70-80% the colonies versus untreated controls (only a 25% drop or so). Here, it can be seen that M1-high4E specimen with the methyltransferase inhibitor (e.g., azacytidine) alone was about 80% of the level of untreated cells, but the combination with ribavirin led to a dramatic reduction to 28% of the level of untreated cells. Ribavirin alone was 43%, and thus the cells respond to the combination better than with either drug alone. These studies show that the response to ribavirin and to the combination of azacytidine plus ribavirin is not restricted to specific lineages (such as the monocytic lineage usually considered for M4 and M5 AML), but rather to whether or not the cells are eIF4E sensitive as typically seen when eIF4E levels are elevated. These studies strongly suggest that any leukemia or other cancer with elevated eIF4E would similarly benefit from the combination.

From Figure 6 and 7, it can be noted that the inhibitor of eIF4E, more particularly ribavirin, inhibits eIF4E activity in azacytidine treated samples, suggesting that targeting the eIF4E pathways in this context is worthwhile.

In order to assess the molecular effects, the Applicant has examined the effects of an inhibitor of eIF4E, e.g., ribavirin, and a methyltransferase inhibitor, e.g., azacytidine, treatment relative to other combinations both by colony assay in primary specimens (see Figure 1) or in AML cells lines characterized by elevated eIF4E levels (see Figures 4 to 7). These cell lines were purchased from the ATCC and are known as KG-1a and THP-1 cells. The Applicant was able to demonstrate that they had not only elevated elF4E levels (see Figure 4) but also nuclear accumulations of eIF4E typical of M4 and M5 AML patients. Thus, these cell lines were a good model. By western blot analysis, the Applicant observed that the inhibitor of eIF4E, e.g., ribavirin, reduced the expression of many known eIF4E targets such as mdm2 (so increased p53 protein), XIAP and Mcl-1 (all anti-apoptotic proteins) (see Figures 5 to 7). In addition, the Applicant noted that the methyltransferase inhibitor, e.g., azacytidine alone led to a gross increase of these eIF4E targets particularly in KG1 cells, relative to any other treatment we used or to untreated cells (see Figure 6). The addition of an inhibitor of eIF4E, e.g., ribavirin, to a methyltransferase inhibitor, e.g., azacytidine, led to a substantial reduction in eIF4E target levels, which strongly indicated that the methyltransferase inhibitor, e.g., azacytidine, stimulates eIF4E pathways (perhaps as a compensatory mechanism in some cells), and the addition of an inhibitor of eIF4E, e.g., ribavirin, to this regimen will be key to the long term success of the methyltransferase inhibitor, e.g., azacytidine, treatment in patients with elevated eIF4E. This correlates with the physiological effects we observed in patient specimens.

The combination according to the present invention also improves response relative to ribavirin alone. As described above, azacytidine potentiates ribavirin activity likely by inhibiting parallel pathways which ribavirin does not target, by potentiating the p53 mediated cell death and others (see figure 8). For instance, ribavirin reduces mdm2 protein levels (Figure 5), and azacytidine increases p53 levels (Weiland 2009, J Pharmacology and Expt Therapeutics). Thus, the net result is a more potent cell death pathway. Further, ribavirin induces a G1/S cell cycle arrest, whereas azacytidine mediated cell death is preferential to G1. In this case, the cell death activity of azacytidine is key to success given that ribavirin induces cell cycle arrest (is cytostatic) rather than cytotoxic, like azacytidine. Finally, the Applicant has observed increased markers for apoptosis and for autophagy upon combination of these drugs in our tissue culture cells. This is observed by increased caspase 3 cleavage for apoptosis and increased LC3B cleavage for autophagy. Interestingly, the inhibitor of eIF4E, e.g., ribavirin, alone elevated LC3B levels versus control KG-1a cells and azacytidine further potentiated that (as seen by increased LC3B cleavage in untreated versus ribavirin/azaycytidine treated cells). Thus, an inhibitor of eIF4E, e.g., ribavirin, alone or in combination with a methyltransferase inhibitor, e.g., azacytidine, leads to multiple types of cell death (not only apoptotic death) and that together this more efficiently inhibits colony growth in the primary AML specimens as observed by the Applicant in Figure 1.

## Claims

1. A pharmaceutical composition comprising a ribavirin, a methyltransferase inhibitor and a pharmaceutically acceptable carrier for use in the treatment of a pre-neoplasm, a precancerous lesion, neoplasm or a proliferative disorder, wherein the methyltransferase inhibitor is azacytidine.

2. The pharmaceutical composition comprising ribavirin and methyltransferase inhibitor for use according to claim 1, wherein the ribavirin and the methyltransferase inhibitor are present in a ratio ranging from 1:5 to 5:1.

3. The pharmaceutical composition comprising ribavirin and methyltransferase inhibitor for use according to claim 1, wherein the ribavirin and methyltransferase inhibitor are present in a ratio ranging from 1:3 to 3:1.

4. The pharmaceutical composition comprising ribavirin and methyltransferase inhibitor for use according to any one of claims 1 to 3, wherein the neoplasm is a cancer.

5. The pharmaceutical composition comprising ribavirin and methyltransferase inhibitor for use according to claim 4, wherein said cancer is selected from the group consisting of: leukemia, acute myeloid leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia, polycythemia vera, lymphoma, Hodgkin's disease, non-Hodgkin's disease lymphoma, Waldenstrom's macroglobulinemia, heavy chain disease, fibrosarcoma, myxosarcoma, liposarcoma, chondrosarcoma, osteogenic sarcoma, chordoma, angiosarcoma, endotheliosarcoma, lyinphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, retinoblastoma, lung cancer, squamous cell carcinoma, adenocarinoma, large cell carcinoma, colorectal cancer, ovarian cancer, ovarian adenocarcinoma, prostate cancer, myelodysplastic syndromes, and multiple myeloma.

6. The pharmaceutical composition comprising ribavirin and methyltransferase inhibitor for use according to claim 5, wherein the acute myeloid leukemia is acute myeloid leukemia M4 or acute myeloid leukemia M5 or another AML subtype **characterized by** atypical elevation of eIF4E.

7. The pharmaceutical composition comprising ribavirin and methyltransferase inhibitor for use according to claim 1, wherein the pre-neoplasm or pre-cancerous lesion is any of the family of proliferative disorders that lead to the development of solid or hematological neoplasms.

8. The pharmaceutical composition comprising ribavirin and methyltransferase inhibitor for use according to any one of claims 1 to 7, wherein said composition is suitable for inhalation, ocular administration, nasal instillation, parenteral administration, dermal administration, transdermal administration, buccal administration, rectal administration, sublingual administration, perilingual administration, nasal administration, topical administration, or oral administration.

9. The pharmaceutical composition comprising ribavirin and methyltransferase inhibitor for use according to any one of claims 1 to 8, wherein said composition is in a unit dosage form.

10. The pharmaceutical composition comprising ribavirin and methyltransferase inhibitor for use according to any one of claims 1 to 9, wherein the ribavirin and the methyltransferase inhibitor are sequentially or simultaneously co-administered.

11. The pharmaceutical composition comprising ribavirin and methyltransferase inhibitor for use according to any one of claims 1 to 10, wherein said composition is present in an amount effective for treating a neoplasm, cancer, acute myeloid leukemia, pre-neoplasm or a pre-cancerous lesion.

12. The pharmaceutical composition comprising ribavirin and methyltransferase inhibitor for use according to any one of claims 1 to 11, wherein the ribavirin is administered in an amount between 500 to 4400 mg per day and the methyltransferase inhibitor is administered in an amount ranging between 50 to 150 mg/m², for up to 7 days every 4 weeks.

13. The pharmaceutical composition comprising ribavirin and methyltransferase inhibitor for use according to claim 12, wherein the ribavirin is administered in an amount between 1000 to 2800 mg per day and the methyltransferase inhibitor is administered in an amount ranging up to 100 mg/m², for up to 7 days every 4 weeks.

14. The pharmaceutical composition comprising ribavirin and methyltransferase inhibitor for use according to any one of claims 1 to 13, wherein the methyltransferase inhibitor is administered in an amount sufficient to repress tumorgenicity of cells.

15. The pharmaceutical composition comprising ribavirin and methyltransferase inhibitor for use according to any one of claims 1 to 13, wherein the methyltransferase inhibitor is administered in a low dose.

16. The pharmaceutical composition comprising ribavirin and methyltransferase inhibitor for use according to claim 15, wherein the low dose ranges from 5 to 15 nM.

17. The pharmaceutical composition comprising ribavirin and methyltransferase inhibitor for use according to any one of claims 1 to 16, further comprising at least one other pharmaceutically active substance, wherein said at least one other pharmaceutically active substance is selected from the group consisting of: topoisomerase inhibitors, NFκB inhibitors, anthracyclines and cisplatin.

18. The pharmaceutical composition comprising ribavirin and methyltransferase inhibitor for use according to claim 17, wherein the topoisomerase inhibitors are selected from the group consisting of: etoposide, topotecan, camptothecan, hycaptamine, irinotecan, rubitecan, 6-ethoxypropionyl-3',4'-O-exo-benzylidene-chartreusin, 9-methoxy-N,N-dimethyl-5-nitropyrazolo[3,4,5-kl]acridine-2-(6H) propanamine, 1-amino-9-ethyl-5-fluoro-2,3-dihydro-9-hydroxy-4-methyl-1H,12H-benzo[de]pyrano[3',4':b,7]-indolizino[1,2b]quinoline-10,13(9H, 15H)dione, lurtotecan, 7-[2-(N-isopropylamino)ethyl]-(20S)camptothecin, BNP1350, BNPI1100, BN80915, BN80942, etoposide phosphate, teniposide, sobuzoxane, 2'-dimethylamino-2'-deoxy-etoposide, GL331, N-[2-(dimethylamino)ethyl]-9-hydroxy-5,6-dimethyl-6H-pyrido[4,3-b]carbazole-1-carboxamide, asulacrine, (5a, saB, 8aa,9b)-9-[2-[N-[2-(dimethylamino)ethyl]-N-methylamino]ethyl]-5-[4-hydrox-y-3,5-dimethoxyphenyl]-5,5a,6,8,8a,9-hexohydrofuro(3',4':6,7)naphtho(2,3-d-)-1,3-dioxol-6-one, 2,3-(methylenedioxy)-5-methyl-7-hydroxy-8-methoxybenzo[c]-phenanthradinium, 6,9-bis[(2-aminoethyl)amino]benzo[g]isoquinoline-5,10-dione, 5-(3-aminopropylamino)-7,10-dihydroxy-2-(2-hydroxyethylaminomethyl)-6H-pyrazolo[4,5,1-de]acridin-6-one, N-[1-[2(diethylamino)ethylamino]-7-methoxy-9-oxo-9H-thioxanthene-4-ylmethyl]formamide, N-(2-(dimethylamino)ethyl)acridine-4-carboxamide, 6-[[2-(dimethylamino)ethyl]amino]-3-hydroxy-7H-indeno[2,1-c]quinolin-7-one, dimesna, and their derivatives.

19. The pharmaceutical composition comprising ribavirin and methyltransferase inhibitor for use according to claim 17 or 18, wherein the NFκB inhibitor is selected from the group consisting of: natural antioxidants and synthetic NFκB inhibitors.

20. The pharmaceutical composition comprising ribavirin and methyltransferase inhibitor for use according to claim 19, wherein the natural antioxidants are selected from the group consisting of: isoflavone genistein, indole-3-carbinol (13C), 3,3'-diindolylmethane (DIM), curcumin, (-)-epigallocatechin-3-gallate (EGCG), resveratrol, lycopene, vitamin E, vitamin C, and combinations thereof.

21. The pharmaceutical composition comprising ribavirin and methyltransferase inhibitor for use according to claim 19 or 20, wherein the synthetic NFκB inhibitors are selected from the group consisting of: dehydroxymethylpoxyquinomicin, cyclooxygenase-2-inhibitors, parthenolide, sulfasalazine, proteasome inhibitors, (E)-3-(4-Methylphenylsulfonyl)-2-propenenitrile (BAY 11-7082), N-[3,5-Bis(trifluoromethyl)phenyl]-5-chloro-2-hydroxybenzamide (IMD-0354), SAHA and combinations thereof.

22. The pharmaceutical composition comprising ribavirin and methyltransferase inhibitor for use according to claim 21, wherein the proteasome inhibitors are selected from the group consisting of: PS-341 and MG-132.

23. The pharmaceutical composition comprising ribavirin and methyltransferase inhibitor for use according to claim 22, wherein PS-341 is bortezomib.

24. The pharmaceutical composition comprising ribavirin and methyltransferase inhibitor for use according to any one of claims 17 to 23, wherein the anthracyclines are selected from the group consisting of: daunorubicin, doxorubicin, epirubicin, idarubicin, and combinations thereof.

## Patentansprüche

1. Pharmazeutische Zusammensetzung, umfassend ein Ribavirin, einen Methyltransferase-Inhibitor und einen pharmazeutisch unbedenklichen Träger zur Verwendung bei der Behandlung eines Präneoplasmas, einer präkanzerösen Läsion, eines Neoplasmas oder einer proliferativen Erkrankung, wobei es sich bei dem Methyltransferase-Inhibitor um Azacytidin handelt.

2. Pharmazeutische Zusammensetzung mit Ribavirin und Methyltransferase-Inhibitor zur Verwendung nach Anspruch 1, wobei das Ribavirin und der Methyltransferase-Inhibitor in einem Verhältnis im Bereich von 1:5 bis 5:1 vorhanden sind.

3. Pharmazeutische Zusammensetzung mit Ribavirin und Methyltransferase-Inhibitor zur Verwendung nach Anspruch 1, wobei das Ribavirin und der Methyltransferase-Inhibitor in einem Verhältnis im Bereich von 1:3 bis 3:1 vorhanden sind.

4. Pharmazeutische Zusammensetzung mit Ribavirin und Methyltransferase-Inhibitor zur Verwendung nach einem der Ansprüche 1 bis 3, wobei es sich bei dem Neoplasma um ein Karzinom handelt.

5. Pharmazeutische Zusammensetzung mit Ribavirin und Methyltransferase-Inhibitor zur Verwendung nach Anspruch 4, wobei das Karzinom ausgewählt ist aus der Gruppe bestehend aus: Leukämie, akuter myeloischer Leukämie, akuter Myelozytenleukämie, akuter myeloblastischer Leukämie, akuter Promyelozytenleukämie, akuter Myelomonozytenleukämie, akuter Monozytenleukämie, akuter Erythroleukämie, chronischer Leukämie, chronischer Myeolzytenleukämie, chronischer Lymphozytenleukämie, Polycythaemia vera, Lymphom, Hodgkin-Krankheit, Non-Hodgkin-Lymphom, Waldenströmscher Makroglobulinämie, Schwerekettenkrankheit, Fibrosarkom, Myxosarkom, Liposarkom, Chondrosarkom, Osteosarkom, Chordom, Angiosarkom, Endothelsarkom, Lymphangiosarkom, Lymphangioendotheliosarkom, Synoviom, Mesotheliom, Ewing-Tumor, Leiomyosarkom, Rhabdomyosarkom, Dickdarmkarzinom, Pankreaskarzinom, Brustkrebs, Ovarialkarzinom, Prostatakrebs, Plattenepithelkarzinom, Basalzellenkarzinom, Adenokarzinom, Schweißdrüsenkarzinom, Talgdrüsenkarzinom, Papillenkarzinom, papillärem Adenokarzinom, Zystadenokarzinom, medullärem Karzinom, Bronchialkarzinom, hypemephroidem Karzinom, Hepatom, Gallengangskarzinom, Choriokarzinom, Seminom, Embryonalkarzinom, Wilms-Tumor, Zervixkarzinom, Gebärmutterkrebs, Hodenkrebs, Lungenkarzinom, kleinzelligem Lungenkarzinom, Blasenkrebs, epithelialem Karzinom, Gliom, Astrozytom, Medulloblastom, Kraniopharyngiom, Ependymom, Pinealom, Hämangioblastom, Akustikusneurinom, Oligodendrogliom, Schwannom, Meningiom, Melanom, Neuroblastom, Retinoblastom, Lungenkrebs, Plattenepithelkarzinom, Adenokarzinom, großzelligem Karzinom, kolorektalem Karzinom, Eierstockkrebs, Ovarialadenokarzinom, Prostatakrebs, myelodysplastischen Syndromen und multiplem Myelom.

6. Pharmazeutische Zusammensetzung mit Ribavirin und Methyltransferase-Inhibitor zur Verwendung nach Anspruch 5, wobei es sich bei der akuten myeloischen Leukämie um akute myeloische Leukämie M4 oder akute myeloische Leukämie M5 oder einen anderen AML-Subtyp handelt, der durch atypische Erhöhung von eIF₄E gekennzeichnet ist.

7. Pharmazeutische Zusammensetzung mit Ribavirin und Methyltransferase-Inhibitor zur Verwendung nach Anspruch 1, wobei es sich bei dem Präneoplasma oder der präkanzerösen Läsion um eine aus der Familie von proliferativen Erkrankungen handelt, die zur Entwicklung von soliden oder hämatologischen Neoplasmen führen.

8. Pharmazeutische Zusammensetzung mit Ribavirin und Methyltransferase-Inhibitor zur Verwendung nach einem der Ansprüche 1 bis 7, wobei die Zusammensetzung geeignet ist für Inhalation, okulare Verabreichung, nasale Instillation, parenterale Verabreichung, dermale Verabreichung, transdermale Verabreichung, bukkale Verabreichung, rektale Verabreichung, sublinguale Verabreichung, perilinguale Verabreichung, nasale Verabreichung, topische Verabreichung oder orale Verabreichung.

9. Pharmazeutische Zusammensetzung mit Ribavirin und Methyltransferase-Inhibitor zur Verwendung nach einem der Ansprüche 1 bis 8, wobei die Zusammensetzung in Einheitsdosisform ist.

10. Pharmazeutische Zusammensetzung mit Ribavirin und Methyltransferase-Inhibitor zur Verwendung nach einem der Ansprüche 1 bis 9, wobei das Ribavirin und der Methyltransferase-Inhibitor nacheinander oder gleichzeitig miteinander verabreicht werden.

11. Pharmazeutische Zusammensetzung mit Ribavirin und Methyltransferase-Inhibitor zur Verwendung nach einem der Ansprüche 1 bis 10, wobei die Zusammensetzung in einer zur Behandlung eines Neoplasmas, eines Karzinoms, einer akuten myeloischen Leukämie, eines Präneoplasmas oder einer präkanzerösen Läsion wirksamen Menge vorhanden ist.

12. Pharmazeutische Zusammensetzung mit Ribavirin und Methyltransferase-Inhibitor zur Verwendung nach einem der Ansprüche 1 bis 11, wobei das Ribavirin in einer Menge von 500 bis 4400 mg pro Tag verabreicht wird, und der Methyltransferase-Inhibitor in einer Menge im Bereich von 50 bis 150 mg/m² für bis zu 7 Tage alle 4 Wochen verabreicht wird.

13. Pharmazeutische Zusammensetzung mit Ribavirin und Methyltransferase-Inhibitor zur Verwendung nach Anspruch 12, wobei das Ribavirin in einer Menge von 1000 bis 2800 mg pro Tag verabreicht wird, und der Methyltransferase-Inhibitor in einer Menge im Bereich von bis zu 100 mg/m² für bis zu 7 Tage alle 4 Wochen verabreicht wird.

14. Pharmazeutische Zusammensetzung mit Ribavirin und Methyltransferase-Inhibitor zur Verwendung nach einem der Ansprüche 1 bis 13, wobei der Methyltransferase-Inhibitor in einer zur Unterdrückung vom Tumorgenizität von Zellen ausreichenden Menge verabreicht wird.

15. Pharmazeutische Zusammensetzung mit Ribavirin und Methyltransferase-Inhibitor zur Verwendung nach einem der Ansprüche 1 bis 13, wobei der Methyltransferase-Inhibitor in einer niedrigen Dosis verabreicht wird.

16. Pharmazeutische Zusammensetzung mit Ribavirin und Methyltransferase-Inhibitor zur Verwendung nach Anspruch 15, wobei die niedrigen Dosen im Bereich von 5 bis 15 nM liegen.

17. Pharmazeutische Zusammensetzung mit Ribavirin und Methyltransferase-Inhibitor zur Verwendung nach einem der Ansprüche 1 bis 16, ferner umfassend mindestens eine andere pharmazeutische aktive Substanz, wobei die mindestens eine andere pharmazeutisch aktive Substanz ausgewählt ist aus der Gruppe bestehend aus: Topoisomerase-Inhibitoren, NF_{K}B -Inhibitoren, Anthracyclinen und Cisplatin.

18. Pharmazeutische Zusammensetzung mit Ribavirin und Methyltransferase-Inhibitor zur Verwendung nach Anspruch 17, wobei die Topoisomerase-Inhibitoren ausgewählt sind aus der Gruppe bestehend aus: Etoposid, Topotecan, Camptothecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H, 12H-benzo[de]pyrano[3',4':b,7]-indolizino[1,2b]chinolin-10,13(9H, 15H)dion, Lurtotecan, 7-[2-(N-Isopropylamino)ethyl]-(20S)camptothecin, BNP1350, BNPI1100, BN80915, BN80942, Etoposidphosphat, Teniposid, Sobuzoxan, 2'-Dimethylamino-2'-deoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydrox-y-3,5-di methoxyphenyl]-5,5a,6,8,8a,9-hexohydrofuro(3',4':6,7)naphtho(2,3-d-)-1,3-dioxol-6-on, 2,3-(Methylendioxy)-5-methyl-7-hydroxy-8-methoxybenzo[c]-phenanthradinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-d e]acridin-6-on, N-[1-[2-(Diethylamino)ethylamino]-7-methoxy-9-oxo-9H-thioxanthen-4-ylmethyl]formamid, N-(2-(Dimethylamino)ethyl)acridin-4-carboxamid, 6-[[2-(Dimethylamino)ethyl]amino]-3-hydroxy-7H-indeno[2,1-c]chinolin-7-on, Dimesna und ihren Derivaten.

19. Pharmazeutische Zusammensetzung mit Ribavirin und Methyltransferase-Inhibitor zur Verwendung nach Anspruch 17 oder 18, wobei der NF_{K}B-Inhibitor ausgewählt ist aus der Gruppe bestehend aus: natürlichen Antioxidanzien und synthetischen NF_{K}B-Inhibitoren.

20. Pharmazeutische Zusammensetzung mit Ribavirin und Methyltransferase-Inhibitor zur Verwendung nach Anspruch 19, wobei die natürlichen Antioxidanzien ausgewählt sind aus der Gruppe bestehend aus: Isoflavongenistein, Indol-3-carbinol (13C), 3,3'-Diindolylmethan (DIM), Curcumin, (-)-Epigallocatechin-3-gallat (EGCG), Resveratrol, Lycopen, Vitamin E, Vitamin C und Kombinationen davon.

21. Pharmazeutische Zusammensetzung mit Ribavirin und Methyltransferase-Inhibitor zur Verwendung nach Anspruch 19 oder 20, wobei die synthetischen NF_{K}B-Inhibitoren ausgewählt sind aus der Gruppe bestehend aus: Dehydroxymethylpoxyquinomicin, Cyclooxygenase-2-Inhibitoren, Parthenolid, Sulfasalazin, Proteasom-Inhibitoren, (E)-3-(4-Methylphenylsulfonyl)-2-propennitril (BAY 11-7082), N-[3,5-Bis(trifluormethyl)phenyl]-5-chlor-2-hydroxybenzamid (IMD-0354), SAHA und Kombinationen davon.

22. Pharmazeutische Zusammensetzung mit Ribavirin und Methyltransferase-Inhibitor zur Verwendung nach Anspruch 21, wobei die Proteasom-Inhibitoren ausgewählt sind aus der Gruppe bestehend aus: PS-341 und MG-132.

23. Pharmazeutische Zusammensetzung mit Ribavirin und Methyltransferase-Inhibitor zur Verwendung nach 22, wobei es sich bei PS-341 um Bortezomib handelt.

24. Pharmazeutische Zusammensetzung mit Ribavirin und Methyltransferase-Inhibitor zur Verwendung nach Anspruch 17 oder 23, wobei die Anthracycline ausgewählt sind aus der Gruppe bestehend aus: Daunorubicin, Doxorubicin, Epirubicin, Idarubicin und Kombinationen davon.

## Revendications

1. Composition pharmaceutique comprenant une ribavirine, un inhibiteur de méthyltransférase et un vecteur de qualité pharmaceutique, destinée au traitement d'un prénéoplasme, d'une lésion précancéreuse, d'un néoplasme ou d'un trouble prolifératif, dans laquelle l'inhibiteur de méthyltransférase est l'azacytidine.

2. Composition pharmaceutique comprenant une ribavirine et un inhibiteur de méthyltransférase pour une utilisation selon la revendication 1, dans laquelle la ribavirine et l'inhibiteur de méthyltransférase sont présents dans un rapport qui s'étend de 1:5 à 5:1.

3. Composition pharmaceutique comprenant une ribavirine et un inhibiteur de méthyltransférase pour une utilisation selon la revendication 1, dans laquelle la ribavirine et l'inhibiteur de méthyltransférase sont présents dans un rapport qui s'étend de 1:3 à 3:1.

4. Composition pharmaceutique comprenant une ribavirine et un inhibiteur de méthyltransférase pour une utilisation selon l'une quelconque des revendications 1 à 3, dans laquelle le néoplasme est un cancer.

5. Composition pharmaceutique comprenant une ribavirine et un inhibiteur de méthyltransférase pour une utilisation selon la revendication 4, dans laquelle ledit cancer est sélectionné dans le groupe constitué par : leucémie, leucémie myéloïde aiguë, leucémie myélocytique aiguë, leucémie myéloblastique aiguë, leucémie promyélocytaire aiguë, leucémie myélomonocytaire aiguë, leucémie monocytaire aiguë, érythroleucémie aiguë, leucémie chronique, leucémie myélogène chronique, leucémie lymphocyte chronique, maladie de Vaquez, lymphome, maladie de Hodgkin, lymphome non hodgkinien, macroglobulinémie de Waldenstrom, maladie des chaînes lourdes, fibrosarcome, myxosarcome, liposarcome, chondrosarcome, sarcome ostéogénique, chordome, angiosarcome, endothéliosarcome, lyinphangiosarcome, lymphangioendothéliosarcome, synoviome, mésothéliome, tumeur d'Ewing, léiomyosarcome, rhabdomyosarcome, carcinome du côlon, cancer du pancréas, cancer du sein, cancer de l'ovaire, cancer de la prostate, carcinome squameux, carcinome basocellulaire, adénocarcinome, adénocarcinome sudoripare, carcinome des glandes sébacées, carcinome papillaire, adénocarcinomes papillaires, cystadénocarcinome, carcinome médullaire, carcinome broncho-pulmonaire, carcinome rénal, hépatome, carcinome du conduit biliaire, choriocarcinome, séminome, carcinome embryonnaire, tumeur de Wilm, cancer du col utérin, cancer de l'utérus, cancer du testicule, carcinome pulmonaire, carcinome bronchique à petites cellules, carcinome de la vessie, carcinome épithélial, gliome, astrocytome, médulloblastome, craniopharyngiome, épendymome, pinéalome, hémangioblastome, neurinome acoustique, oligodendrogliome, schwannome, méningiome, mélanome, neuroblastome, rétinoblastome, cancer du poumon, carcinome squameux, adénocarcinome, carcinome à grandes cellules, cancer colorectal, cancer de l'ovaire, adénocarcinome ovarien, cancer de la prostate, syndromes myélodysplasiques et myélome multiple.

6. Composition pharmaceutique comprenant une ribavirine et un inhibiteur de méthyltransférase pour une utilisation selon la revendication 5, dans laquelle la leucémie myéloïde aiguë est une leucémie myéloïde aiguë M4 ou une leucémie myéloïde aiguë M5 ou un autre sous-type de LMA **caractérisée par** une élévation atypique de eIF₄E.

7. Composition pharmaceutique comprenant une ribavirine et un inhibiteur de méthyltransférase pour une utilisation selon la revendication 1, dans laquelle le prénéoplasme ou la lésion précancéreuse est l'un quelconque de la famille de troubles prolifératifs qui mènent au développement de néoplasmes solides ou hématologiques.

8. Composition pharmaceutique comprenant une ribavirine et un inhibiteur de méthyltransférase pour une utilisation selon l'une quelconque des revendications 1 à 7, dans laquelle ladite composition est appropriée pour inhalation, administration oculaire, instillation nasale, administration parentérale, administration dermique, administration transdermique, administration buccale, administration rectale, administration sublinguale, administration perlinguale, administration nasale, administration topique et administration orale.

9. Composition pharmaceutique comprenant une ribavirine et un inhibiteur de méthyltransférase pour une utilisation selon l'une quelconque des revendications 1 à 8, dans laquelle ladite composition est sous une forme de dose unitaire.

10. Composition pharmaceutique comprenant une ribavirine et un inhibiteur de méthyltransférase pour une utilisation selon l'une quelconque des revendications 1 à 9, dans laquelle la ribavirine et l'inhibiteur de méthyltransférase sont séquentiellement ou simultanément coadministrés.

11. Composition pharmaceutique comprenant une ribavirine et un inhibiteur de méthyltransférase pour une utilisation selon l'une quelconque des revendications 1 à 10, dans laquelle ladite composition est présente en une quantité efficace pour le traitement d'un néoplasme, d'un cancer, d'une leucémie myéloïde aiguë, d'un prénéoplasme ou d'une lésion précancéreuse.

12. Composition pharmaceutique comprenant une ribavirine et un inhibiteur de méthyltransférase pour une utilisation selon l'une quelconque des revendications 1 à 11, dans laquelle la ribavirine est administrée en une quantité entre 500 et 4400 mg par jour et l'inhibiteur de méthyltransférase est administré en une quantité dans la plage entre 50 et 150 mg/m², pendant jusqu'à sept jours toutes les quatre semaines.

13. Composition pharmaceutique comprenant une ribavirine et un inhibiteur de méthyltransférase pour une utilisation selon la revendication 12, dans laquelle la ribavirine est administrée en une quantité entre 1000 et 2800 mg par jour et l'inhibiteur de méthyltransférase est administré en une quantité allant jusqu'à 100 mg/m², pendant jusqu'à sept jours toutes les quatre semaines.

14. Composition pharmaceutique comprenant une ribavirine et un inhibiteur de méthyltransférase pour une utilisation selon l'une quelconque des revendications 1 à 13, dans laquelle l'inhibiteur de méthyltransférase est administré en une quantité suffisante pour réprimer le potentiel tumorigène des cellules.

15. Composition pharmaceutique comprenant une ribavirine et un inhibiteur de méthyltransférase pour une utilisation selon l'une quelconque des revendications 1 à 13, dans laquelle l'inhibiteur de méthyltransférase est administré à une faible dose.

16. Composition pharmaceutique comprenant une ribavirine et un inhibiteur de méthyltransférase pour une utilisation selon la revendication 15, dans laquelle la faible dose varie de 5 à 15 nM.

17. Composition pharmaceutique comprenant une ribavirine et un inhibiteur de méthyltransférase pour une utilisation selon l'une quelconque des revendications 1 à 16, comprenant en outre au moins une autre substance pharmaceutiquement active, dans laquelle ladite au moins une autre substance pharmaceutiquement active est sélectionnée dans le groupe constitué par : inhibiteurs de topoisomérase, inhibiteurs de NFKB, anthracyclines et cisplatine.

18. Composition pharmaceutique comprenant une ribavirine et un inhibiteur de méthyltransférase pour une utilisation selon la revendication 17, dans laquelle les inhibiteurs de topoisomérase sont sélectionnés dans le groupe constitué par : étoposide, topotécan, camptothécan, hycaptamine, irinotécan, rubitécan, 6-éthoxypropionyl-3',4'-O-exo-benzylidène-chartreusine, 9-méthoxy-N,N-diméthyl-5-nitropyrazolo[3,4,5-kl]acridine-2-(6H) propanamine, 1-amino-9-éthyl-5-fluoro-2,3-dihydro-9-hydroxy-4-méthyl-1H,12H-benzo[de]pyrano[3'4':b,7]-i ndolizino[1,2b]quinoline-10,13(9H, 15H)dione, lurtotécan, 7-[2-(N-isopropylamino)éthyl]-(20S)camptothécine, BNP1350, BNPI1100, BN80915, BN80942, phosphate d'étoposide, téniposide, sobuzoxane, 2'-diméthylamino-2'-déoxy-étoposide, GL331, N-[2-(diméthylamino)éthyl]-9-hydroxy-5,6-diméthyl-6H-pyrido[4,3-b]carbazole-1-carboxamide, asulacrine, (5a,5aB,8aa,9b)-9-[2-[N-[2-(diméthylamino)éthyl]-N-méthylamino]éthyl]-5-[4-hydrox-y-3,5-di méthoxyphényl]-5,5a,6,8,8a,9-hexohydrofuro(3',4':6,7)naphto(2,3-d-)-1,3-dioxol-6-one, 2,3-(méthylenedioxy)-5-méthyl-7-hydroxy-8-méthoxybenzo[c]-phénanthradinium, 6,9-bis[(2-aminoéthyl)amino]benzo[g]isoquinoline-5,10-dione, 5-(3-aminopropylamino)-7,10-dihydroxy-2-(2-hydroxyéthylaminométhyl)-6H-pyrazolo[4,5,1-de ]acridin-6-one, N-[1-[2(diéthylamino)éthylamino]-7-méthoxy-9-oxo-9H-thioxanthène-4-ylméthyl]formamide, N-(2-(diméthylamino)éthyl)acridine-4-carboxamide, 6-[[2-(diméthylamino)éthyl]amino]-3-hydroxy-7H-indéno[2,1-c]quinolin-7-one, dimesna et leurs dérivés.

19. Composition pharmaceutique comprenant une ribavirine et un inhibiteur de méthyltransférase pour une utilisation selon la revendication 17 ou 18, dans laquelle l'inhibiteur de NFKB est sélectionné dans le groupe constitué par : antioxydants naturels et inhibiteurs de NFKB de synthèse.

20. Composition pharmaceutique comprenant une ribavirine et un inhibiteur de méthyltransférase pour une utilisation selon la revendication 19, dans laquelle les antioxydants naturels sont sélectionnés dans le groupe constitué par : isoflavone génistéine, indole-3-carbinol (13 C), 3,3'-diindolylméthane (DIM), curcumine, (-)-épigallocatéchin-3-gallate (EGCG), resvératrol, lycopène, vitamine E, vitamine C et combinaisons de ceux-ci.

21. Composition pharmaceutique comprenant une ribavirine et un inhibiteur de méthyltransférase pour une utilisation selon la revendication 19 ou 20, dans laquelle les inhibiteurs de NFKB de synthèse sont sélectionnés dans le groupe constitué par : déhydroxyméthylpoxyquinomicine, cyclooxygénase-2-inhibiteurs, parthénolide, sulfasalazine, inhibiteurs de protéasome, (E)-3-(4-méthylphénylsulfonyl)-2-propènenitrile (BAY 11-7082), N-[3,5-bis(trifluorométhyl)phényl]-5-chloro-2-hydroxybenzamide (IMD-0354), SAHA et combinaisons de ceux-ci.

22. Composition pharmaceutique comprenant une ribavirine et un inhibiteur de méthyltransférase pour une utilisation selon la revendication 21, dans laquelle les inhibiteurs de protéasome sont sélectionnés dans le groupe constitué par : PS-341 et MG-132.

23. Composition pharmaceutique comprenant une ribavirine et un inhibiteur de méthyltransférase pour une utilisation selon la revendication 22, dans laquelle PS-341 est du bortézomib.

24. Composition pharmaceutique comprenant une ribavirine et un inhibiteur de méthyltransférase pour une utilisation selon l'une quelconque des revendications 17 à 23, dans laquelle les anthracyclines sont sélectionnés dans le groupe constitué par : daunorubicine, doxorubicine, épirubicine, idarubicine et combinaisons de celles-ci.
